# EUROPEAN PATENT APPLICATION

(11) **EP 4 596 585 A1**
(43) Date of publication of application: **06.08.2025**
(21) Application number: 23870682.4
(22) Date of filing: 25.09.2023
(51) Int. Cl.: C07K 19/00, C12N 15/62, A61K 38/00, A61K 39/00

(54) **CONSTRUCTION OF ENGINEERED EXTRACELLULAR VESICLE AND USE THEREOF**

(30) Priority: 30.09.2022 CN 202211213321
(71) Applicant: Beijing Echo Pharmaceutical Co., Ltd., Beijing 102609 (CN)
(72) Inventor: ZHAO, Libo, Beijing 102609 (CN); KONG, Guanyi, Beijing 102609 (CN); LI, Hongyan, Beijing 102609 (CN); GUAN, Hongtao, Beijing 102609 (CN); WANG, Jianwu, Beijing 102609 (CN); ZHAO, Hang, Beijing 102609 (CN); YOU, Zhicheng, Beijing 102609 (CN); LIU, Da, Beijing 102609 (CN); LIN, Zhongqing, Beijing 102609 (CN); LI, Zhi, Beijing 102609 (CN); CHEN, Jing, Beijing 102609 (CN)
(74) Representative: Grünecker Patent- und Rechtsanwälte PartG mbB
(86) International application number: PCT/CN2023/120966
(87) International publication number: WO 2024/067451

(57) **Abstract**

The present invention provides an engineered extracellular vesicle, a pharmaceutical composition containing the engineered extracellular vesicle and a use thereof, and further provides a method for constructing the engineered extracellular vesicle. The engineered extracellular vesicle can be used for drug delivery, enrichment of target proteins in extracellular vesicles, or display of target proteins.

## Description

This application claims the priority of Chinese Patent Application No. 202211213321.0, filed with the China National Intellectual Property Administration on September 30, 2022, and titled with "CONSTRUCTION OF ENGINEERED EXTRACELLULAR VESICLE AND USE THEREOF", which is hereby incorporated by reference in its entirety.

### FIELD

The present invention relates to the field of biomedicine, in particular to an engineered extracellular vesicle, a production method and use thereof.

### BACKGROUND

Extracellular vesicles (EVs) are membranous vesicles secreted by cells and capable of being taken up by recipient cells, with a diameter of approximately 30-1000 nm. EVs serve as carriers for the intercellular transport of biological macromolecules such as proteins, RNA, and lipids, and are an important medium for intercellular communication. Since EVs widely exist in various body fluids of the human body, they can be used as a sample source for liquid biopsy and are also considered to be a natural domesticated drug carrier.

EVs can be used as drug carriers to carry and transport drugs, such as polypeptides and proteins with therapeutic effects. In order for the target protein carried by EV to be effective, it is necessary to ensure that the amount of the target protein in EV is enough. However, non-exosomal proteins heterologously expressed in cells are not spontaneously sorted into extracellular vesicles.

According to existing reports, there are currently about 20 proteins reported to be used for loading target proteins into EVs (Theranostics, 2019; 9(4): 1015-1028). Patent applications such as WO2013084000A2, WO2014168548A2, WO2018015535A1, and WO2019040920A1 also describe methods of loading proteins into EVs.

### SUMMARY

During research, the inventor of the present application unexpectedly found that the family of plexins has the function of carrying and enriching the target proteins fused with plexins to extracellular vesicles. Based on this finding, the inventor completed the present invention.

In a first aspect, the present invention provides an engineered extracellular vesicle comprising a fusion protein, wherein the fusion protein comprises a fusion partner, which is a protein located on the extracellular vesicle membrane or a variant thereof, preferably, the fusion protein further comprises a target protein.

The term "extracellular vesicles (EVs)" used herein refers to vesicle-like bodies with a double-layer membrane structure that are shed from the cell membrane or secreted by cells, with a diameter ranging from 40nm to 1000nm. The main forms thereof are microvesicles (MVs) and exosomes (Exs). Extracellular vesicles are widely present in cell culture supernatants and various body fluids (blood, lymph, saliva, urine, semen, milk), carrying a variety of proteins, lipids, DNA, mRNA, miRNA, etc., related to cell origin, and involved in processes such as intercellular communication, cell migration, angiogenesis, and immune regulation. The term "engineered extracellular vesicles" refers to artificially synthesized extracellular vesicles, or extracellular vesicles produced by cells after artificial intervention, or extracellular vesicles produced by cells after genetic engineering. The term "unengineered extracellular vesicles" refers to native, unmodified extracellular vesicles secreted by cells under normal conditions (e.g., physiological conditions).

The protein referred to herein includes variants thereof. The "variant" of the protein refers to a protein having different amino acid sequence from the amino acid sequence of the corresponding naturally occurring protein (sometimes also called wild type) but having the same or similar functions with the wild type protein. Variants of a protein may be derived from the naturally occurring protein through amino acid deletions, additions and/or substitutions, and may also be truncated forms of the natural protein (sometimes referred to as fragments thereof).

The term "fusion partner" used herein refers to a polypeptide fused to a target protein. The fusion partner can be located on the membrane of extracellular vesicles and simultaneously carry the target protein fused therewith to the extracellular vesicles..

In some embodiments of the present invention, the fusion partner is characterized in that: 1) the fusion partner is a type I transmembrane protein; 2) a transmembrane region and adjacent intracellular region of the transmembrane protein comprise an α-helix in a length of 20-200 amino acids, preferably 30-90 amino acids, more preferably 40-80 amino acids, and part or all of the transmembrane region is located in the α-helix; and 3) the extracellular region comprises 1 to 5 IPT domains.

The term "type I transmembrane protein" used herein refers to a single-pass transmembrane protein whose N-terminus is located outside the membrane and whose C-terminus is located inside the membrane. The "adjacent intracellular region" in "transmembrane region and adjacent intracellular region" refers to the intracellular fragment immediately adjacent to the transmembrane region. The term "α-helix (alpha helix)" used herein can refer to one alpha helix or multiple alpha helices, such as a coiled coil domain formed by multiple alpha helices.

The term "IPT (immunoglobulin-like fold, plexins, transcription factors) domain" used herein, also known as TIG (transcription factor immunoglobin) domain, refers to a domain with an immunoglobulin-like fold.

In some embodiments, the fusion partner is selected from members of plexins, such as PLXNA, PLXNB, PLXNC, and PLXND family members, such as PLXNA1, PLXNA2, PLXNA3, PLXNA4, PLXNB1, PLXNB2, PLXNC1, and PLXND1.

The currently known members of the plexin family include PLXNA1 (Plexin A1), PLXNA2, PLXNA3, PLXNA4, PLXNB1, PLXNB2, PLXNC1, PLXND1, etc. Plexins are transmembrane protein receptors for semaphorins. These molecules are involved in many cellular activities related to cell proliferation, adhesion and basement membrane, cell motility and invasion. These proteins are expressed in brain tissue, endocrine system, digestive tract system, respiratory system, bone marrow and lymphatic system, etc.

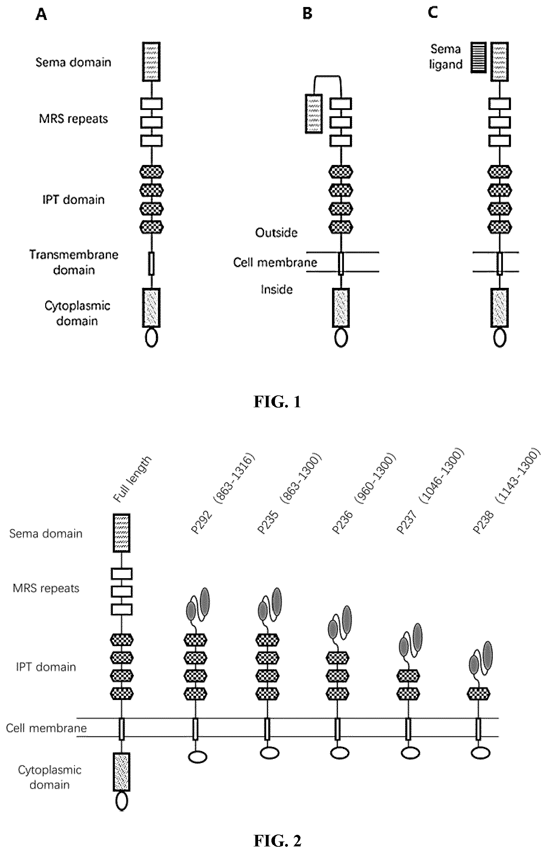

Taking PLXNA1 as an example, PLXNA1 can mediate the remodeling of cytoskeleton that leads to cell migration and axonal repulsion by directly binding to the transmembrane semaphorin 6D, or indirectly binding to type 3 secreted semaphorins through neuropilin-1 and -2. The PLXNA1 protein contains multiple structural domains, as shown in FIG. 1A. Full-length PLXNA1 has two states: activated state (FIG. 1B) and inactive state (FIG. 1C). Since full-length PLXNA1 is biologically active, it is not suitable as a fusion partner for carrying the target protein. The inventor unexpectedly found that by truncating off the extracellular region and/or intracellular region of PLXNA1 and fusing the truncated fragment with the target protein, the target protein can be carried to extracellular vesicles for enrichment.

In particular, the fusion partner is selected from the group consisting of PLXNA1, PLXNA2, and a fragment thereof. The fragment may comprise a fragment having an amino acid sequence selected from the group consisting of SEQ ID NOs: 2-6. In particular, the amino acid sequence the fusion partner is selected from the group consisting of SEQ ID NOs: 2-6.

In some embodiments, the fusion protein is an engineered transmembrane protein expressed from an exogenous sequence, and the transmembrane protein comprises a fragment represented by formula (I):
Xaa₁...Xaaₙ Xaa₍ₙ₊₁₎Xaa₍ₙ₊₂₎ Xaa₍ₙ₊₃₎ Xaa₍ₙ₊₄₎ (I), (SEQ ID NO: 15)
preferably, the fragment represented by formula (I) is located upstream of the transmembrane region;
wherein, Xaa₁ is selected from the group consisting of S, T, C, N, Y and Q; Xaa₂ to Xaaₙ are any amino acids, n is an integer selected from 1 to 20; Xaa₍ₙ₊₁₎, Xaa₍ₙ₊₂₎ and Xaa₍ₙ₊₄₎ are each independently selected from the group consisting of L, I, G, A and V; and Xaa₍ₙ₊₃₎ is selected from the group consisting of T, S, C, N, Y and Q.

In some embodiments, n is 1, 2, 3, 4, 5, 6, 7, 8, 9, 10, 11, 12, 13, 14, 15, 16, 17, 18, 19, or 20, preferably 1, 2, 3, 4, 5, 6, 7, 8, 9 or 10, more preferably 1, 2, 3, 4 or 5. In other embodiments, Xₐₐ₁ is selected from the group consisting of S, T, C, and N; Xaa₍ₙ₊₁₎, Xaa₍ₙ₊₂₎, and Xaa₍ₙ₊₄₎ are each independently selected from the group consisting of L and I; and Xaa₍ₙ₊₃₎ is selected from the group consisting of T, S, C and N. In yet other embodiments, Xₐₐ₁ is S, Xaa₍ₙ₊₁₎, Xaa₍ₙ₊₂₎, and Xaa₍ₙ₊₄₎ are L, and Xaa₍ₙ₊₃₎ is T. In a specific embodiment, the fragment represented by formula (I) is SDSLLTL (SEQ ID NO: 14).

In some embodiments, the fusion protein comprises a sequence selected from SEQ ID NOs: 2-6.

The term "exogenous sequence" used herein means that the sequence is not a naturally occurring sequence in the cell, but an artificially constructed sequence. The amino acids herein may be natural amino acids or unnatural amino acids, represented by three letters or single letters known in the art, for example: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V). "Similar amino acids" refer to some amino acids with similar structures and properties. When these amino acids are replaced with each other, they often have little effect on the structure and properties of the protein and can be used interchangeably.

In some embodiments, the target protein can be located upstream of the fusion partner or downstream of the fusion partner. The "upstream" of a protein used herein refers to the N-terminus of the polypeptide or the part near the N-terminus, and the "downstream" of a protein refers to the C-terminus of the polypeptide or the part near the C-terminus. Depending on actual needs, the target protein can be contained inside the extracellular vesicles or displayed on the outer surface of the extracellular vesicles.

In some embodiments of the present invention, the target protein is a therapeutic peptide, a targeting peptide, an affinity tag, or a linker for linking with a therapeutic compound.

The term "therapeutic peptide" as used herein refers to a protein or a variant thereof with therapeutic activity, including but not limited to antibodies or antigen-binding fragments thereof, receptors, ligands, cytokines, hormones, and the like. In some embodiments of the present invention, the therapeutic peptide may be selected from the group consisting of members of the human interleukin family (e.g., IL-2, IL-7, IL-10, IL-11, IL-12, IL-15, and IL-23), members of the tumor necrosis factor family (e.g., TNF, LTA, LTB, FASLG, TNFSF8, TNFSF9, TNFSF10, TNFSF11, TNFSF12, TNFSF13, TNFSF14, TNFSF15, TNFSF18, and EDA), interferons (INF-α, INF- β and INF-γ), T cell engagers (e.g., 4-1BB, OX40, CD28, CD40, CD40L, CD47, CD27, CD70, CD80, CD86, GITRL, ICOSL, CD155, CD112, TIM-3, BTLA), and other cytokines (e.g., G-CSF, EPO, TPO, GM-CSF, EGF, bFGF, FVIIa, ATIII, TNK, α-Glucosidase, BMP-2, and hirudin).

The term "targeting peptide" as used herein refers to a polypeptide that can be recognized and bound by specific cells, that is, a polypeptide that can target specific cells. In some embodiments of the present invention, the targeting peptide is a polypeptide targeting cells, and the targeting peptide may be an antibody or an antigen-binding fragment thereof, a cell surface receptor, or a ligand.

The term "affinity tag" used herein refers to a short polypeptide that can bind to the corresponding affinity agent and is often used for protein separation and purification. In some embodiments of the present invention, the affinity tag may be selected from the group consisting of His tag, glutathione sulfhydryl transferase (GST), S-peptide, ZZ domain, albumin binding domain (ABD), HA, Myc , FLAGTM, maltose-binding protein (MBP), calmodulin-binding peptide (CBP), SUMO, *Streptococcal* protein G (Protein G) and *Staphylococcus aureus* protein A (Protein A).

As used herein, the term "linker for linking with a therapeutic compound" refers to a substance that links a therapeutic compound to a fusion partner.

In some embodiments of the present invention, the extracellular vesicles comprise two or more fusion proteins, for example, the two or more fusion proteins each independently comprise a different target protein. The different target proteins can form fusion proteins with the same fusion partner, or with different fusion partners, and different fusion proteins can be carried into the same extracellular vesicle.

The term "therapeutic agent" used herein refers to a substance with therapeutic effects, which may be selected from the group consisting of nucleotides, amino acids, lipids, carbohydrates, small molecules, antibodies, enzymes, ligands, receptors, peptides, etc. In some embodiments of the present invention, the extracellular vesicles contain therapeutic agents, which may be therapeutic peptides, polynucleotides, and small molecule compounds.

In a second aspect, the present invention provides a pharmaceutical composition comprising the extracellular vesicle described in the first aspect and a pharmaceutically acceptable carrier.

The term "pharmaceutically acceptable carrier" used herein can be selected from the group consisting of water, buffered aqueous solution, isotonic saline solution such as PBS (phosphate buffer saline), glucose, mannitol, dextrose, lactose, starch, magnesium stearate, cellulose, magnesium carbonate, 0.3% glycerin, hyaluronic acid, ethanol and polyalkylene glycol such as polypropylene glycol and triglyceride. The type of pharmaceutically acceptable carrier used depends especially on whether the composition of the present invention is formulated for oral, nasal, intradermal, subcutaneous, intramuscular or intravenous administration. The composition according to the present invention may comprise a lubricant, a preservative, a stabilizer, a wetting agent, an emulsifier, a salt that influences the osmotic pressure, a buffer, a coloring substance, a flavoring substance and/or an aromatic substance and the like as additives.

In a third aspect, the present invention provides an engineered cell for producing the extracellular vesicle of the first aspect, wherein the cell comprises a polynucleotide encoding the fusion protein.

In some embodiments of the present invention, the polynucleotide encoding the fusion protein is present in the cell in a free manner, for example, introduced into the cell by transient transfection. In other embodiments of the present invention, the polynucleotide is integrated into the genome of the cell. In other embodiments of the present invention, the polynucleotide is of one, two or more types, encoding one, two or more fusion proteins.

In a fourth aspect, the present invention provides a method for treating a disease, comprising administering to a subject in need thereof the extracellular vesicle according to the first aspect, the pharmaceutical composition according to the second aspect, or the cell according to the third aspect.

The term "subject" used herein refers to mammals, such as humans, but can also be other animals, such as wild animals (such as herons, storks and cranes), domestic animals (such as ducks and geese) or experimental animals (such as orangutans, monkeys, rats, mice, rabbits, guinea pigs, groundhogs and ground squirrels).

The compositions of the present invention may be administered by a variety of methods known in the art. Those skilled in the art will understand that the route and/or mode of administration will vary depending on the desired results. In order to administer the compound of the present invention by a particular route of administration, it may be necessary to cover the compound with a material that avoids inactivating the compound, or to co-administer the compound with such material. For example, the compound can be administered to a subject in a suitable carrier, such as liposomes or diluents. Pharmaceutically acceptable diluents include saline solutions and aqueous buffers. Pharmaceutical carriers include sterile aqueous solutions or dispersions and sterile powders for the extemporaneous preparation of sterile injectable solutions or dispersions. The use of such media and agents for pharmaceutically active substances is known in the art.

The present invention further provides use of the extracellular vesicle according to the first aspect, the pharmaceutical composition according to the second aspect, or the cell according to the third aspect in the manufacture of a medicament for treating a disease.

In the fifth aspect, the present invention provides a method for producing the extracellular vesicle according to the first aspect, comprising
1) providing a cell comprising a polynucleotide encoding the fusion protein,
2) culturing the cell under a condition suitable for expression of the fusion protein, and
2) isolating the extracellular vesicle.

In some embodiments of the present invention, the introduction is transient, for example, the polynucleotide encoding the fusion protein is introduced into the host cell by lipofection, electroporation, etc. In other embodiments, the introduction is stable, for example, the polynucleotide encoding the fusion protein is inserted into the genome of the host cell through gene editing technology, so that the host cell stably expresses the engineered extracellular vesicles.

Cells that can be used for protein expression well known to those skilled in the art may be used in the present invention, preferably eukaryotic cells, such as expi293, HEK293 and HEK293F.

In the sixth aspect, the present invention provides a method of delivering a target protein to target cells, comprising
1) providing the extracellular vesicle according to the first aspect, wherein the fusion protein comprises a target protein to be delivered, and
2) contacting the extracellular vesicle with the target cell..

In the seventh aspect, the present invention provides a method of isolating the extracellular vesicle according to the first aspect, comprising
1) expressing the fusion protein in a cell, wherein the fusion protein comprises an affinity tag,
2) contacting the extracellular vesicle with a binding agent capable of binding the affinity tag, and
3) isolating the extracellular vesicle based on the binding of the affinity tag to the binding agent.

The term "binding agent" used herein refers to a substance that can bind to an affinity tag. The binding agent binds to the affinity tag with a high affinity and helps to isolate the fusion protein connected to the affinity tag through the binding agent..

In the eighth aspect, the present invention provides a fusion protein, which is an engineered transmembrane protein expressed by an exogenous sequence, and the transmembrane protein comprises a fragment represented by formula (I):
Xaa₁...Xaaₙ Xaa₍ₙ₊₁₎Xaa₍ₙ₊₂₎Xaa₍ₙ₊₃₎Xaa₍ₙ₊₄₎ (I), (SEQ ID NO: 15)
preferably, the fragment represented by formula (I) is located upstream of the transmembrane region,
wherein, Xaa₁ is selected from the group consisting of S, T, C, N, Y and Q; Xaa₂ to Xaaₙ are any amino acids, n is an integer from 1 to 20; Xaa₍ₙ₊₁₎, Xaa₍ₙ₊₂₎ and Xaa₍ₙ₊₄₎ are each independently selected from the group consisting of L, I, G, A and V; Xaa₍ₙ₊₃₎ is selected from the group consisting of T, S, C, N, Y and Q.

In some embodiments, n is 1, 2, 3, 4, 5, 6, 7, 8, 9, 10, 11, 12, 13, 14, 15, 16, 17, 18, 19, or 20 , preferably 1, 2, 3, 4, 5, 6, 7, 8, 9 or 10, more preferably 1, 2, 3, 4 or 5. In other embodiments, Xₐₐ₁ is selected from the group consisting of S, T, C, and N; Xaa₍ₙ₊₁₎, Xaa₍ₙ₊₂₎, and Xaa₍ₙ₊₄₎ are each independently selected from the group consisting of L and I; Xaa₍ₙ₊₃₎ is selected from the group consisting of T, S, C and N. In yet other embodiments, Xₐₐ₁ is S, Xaa₍ₙ₊₁₎, Xaa₍ₙ₊₂₎, and Xaa₍ₙ₊₄₎ are L, and Xaa₍ₙ₊₃₎ is T. In a specific embodiment, the fragment represented by formula (I) is SDSLLTL (SEQ ID NO: 14).

In some embodiments of the present invention, the fusion protein may comprise a fragment selected from SEQ ID NOs: 2-6.

In the ninth aspect, the present invention provides use of the fusion protein in preparing an engineered extracellular vesicle.

In the tenth aspect, the present invention provides a method of loading a target molecule to the extracellular vesicle of the present invention, comprising contacting the target molecule with the extracellular vesicle, wherein the target molecule and the fusion partner are connected to a part A and a part B respectively, and the part A and part B are bound by affinity. This binding is non-covalent and reversible, and any molecular pairing known in the art that can bind through affinity can be used in the present invention.

The traditional method of producing engineered extracellular vesicles is to first design a plasmid to couple the scaffold protein to the target molecule, then transfect the plasmid into the engineered cells, and then culture the cells under certain conditions to secrete vesicles carrying the target molecules into the supernatant, which are then purified and characterized. This production method is time-consuming, costly, and long-cycle, and is not suitable for personalized loading requirements for different target molecules. In view of the shortcomings of traditional methods such as time consumption, high cost, and long cycle, and to cope with various personalized modification needs, the method of the present invention provides a more general, simple and efficient method for modifying EV to externally load target proteins, and only produces a single type of engineered EV, which is *in vitro* co-incubated with the target protein labeled with specific tags to achieve rapid loading of target proteins.

In some embodiments, the part A and part B are a combination selected from the group consisting of NbALFA/ALFA, biotin/avidin, strepII/streptactin, Intein N/C, SpyCatcher/Spy and Protein A/Fc. The target molecule and the fusion partner are connected to part A and part B respectively. After contacting, the part A and part B are bound by affinity, and the target molecule is loaded on the EV through the fusion partner and displayed on the EV surface.

In some embodiments, the target molecule is a therapeutic peptide, targeting peptide, affinity tag, or linker linking a therapeutic compound. When the target molecule is a polypeptide, the part A or part B can be connected to the N-terminal, C-terminal or interior of the polypeptide as needed. Herein, the target molecule can be a protein of interest, also called POI (protein of interest).

In some embodiments, different target molecules can be loaded on the same EV. For example, different target molecules carry part A, and EVs carry part B. By contacting different target molecules with EVs at the same time, EVs can be loaded with different target molecules at the same time. In addition, the ratio of different target molecules can also be adjusted according to actual needs. For another example, it is also possible to load different target molecules on the same EV in a case that different target molecules carry different types of part A (A1, A2, A3...), and EVs carry different types of part B (B1, B2, B3...).

### BRIEF DESCRIPTION OF DRAWINGS

FIG. 1 shows the structure of PLXNA1 and its different states. A) is a schematic diagram of the structure of PLXNA1; B) shows the inactive state of PLXNA1, with the Sema domain binding to MRS repeats; C) shows the activated state of PLXNA1, with the Sema domain binding to the ligand.
FIG. 2 shows a series of truncated PLXNA1 constructed in Example 3. The N-terminal and C-terminal ends of the truncated PLXNA1 are respectively fused with mIL12 and eGFP.
FIG. 3 is the WB results showing the enrichment of the target protein to EVs by the truncated PLXNA1 constructed in Example 3.
FIG. 4 shows the ELISA for detecting the number of eGFP on EVs produced by truncated PLXNA1 in Example 3.
FIG. 5 shows the comparison of the EV enrichment capacity of PLXNA1 and other EV proteins PTGFRN and Lamp2b in Example 4.
FIG. 6 shows the expression and enrichment of PLXNA2, PLXNB1, PLXNB2, PLXNC1 and PLXND1 on EVs in Example 5.
FIG. 7 shows the copy number of eGFP on each EV in the three samples of P240, P241 and P242.
FIG. 8 shows the sequence alignment of the transmembrane region of PLXNA1 and PLXNA2. The sequences in italics are the last IPT domain close to the transmembrane region, the sequences in bold are the key sequences of 7 amino acids, and the underlined sequences are the transmembrane region.
FIG. 9 shows the impact of key sequences on the EV enrichment effect of fusion proteins. A) is a schematic structural diagram of the P166 and P167 fusion proteins; B) is the WB detection result; C) is the statistical result.
FIG. 10 shows the impact of mutations in the key sequence on the EV enrichment effect of fusion proteins. A) is the result of point mutation at each position; B) is the result of synonymous mutation; C) is the result of insertion mutation.
FIG. 11 shows that different target proteins carried by the truncated PLXNA1 were sorted to EVs.
FIG. 12 shows that mIL12 fused and expressed with truncated PLXNA1 still maintains activity.
FIG. 13 shows the relationship between luciferase activity and EVs particle number.
FIG. 14 is a schematic diagram showing the loading of target protein through intermolecular interaction via the EV fusion partner based on the present invention
FIG. 15 shows the expression of NbALFA-Flag-PLXΔ in EVs detected by WB in Example 11.
FIG. 16 shows the results of nanoflow cytometry detection of NbALFA-PLXΔ-EV in Example 11.
FIG. 17 shows the results of nanoflow cytometry detection of NbALFA-PLXΔ-EV loading GFP in Example 11.
FIG. 18 shows the results of nanoflow cytometry detection of NbALFA-PLXΔ-EV loading RVG polypeptide in Example 11.
FIG. 19 shows the affinity detection results of RVG-labeled vesicles to receptor cell N2a in Example 11.
FIG. 20 shows the results of nanoflow cytometry detection of NbALFA-PLXΔ-EV loading trastuzumab scFv fragment in Example 11.
FIG. 21 shows the affinity detection results of vesicles labeled with trastuzumab scFv fragment to the recipient cell SK-BR-3 in Example 11.
FIG. 22 shows the freeze-thaw cycle test results of EV in Example 11.
FIG. 23 shows the results of nanoflow cytometry detection of the average fluorescence intensity of GFP in the second and fourth weeks when the GFP-labeled NbALFA-PLXΔ-EV was stored at 4°C or -80°C in Example 11.
FIG. 24 shows the results of WB detection of different tags in Example 12.
FIG. 25 shows the expression of mSA-Flag-PLXΔ in EVs detected by WB in Example 13.
FIG. 26 shows the results of nanoflow cytometry detection of mSA-PLXΔ-EV in Example 13.
FIG. 27 shows the results of nanoflow cytometry detection of biotin-488 labeled mSA-PLXΔ-EV in Example 13.
FIG. 28 shows the results of nanoflow cytometry detection of FITC-HA labeled EVs in Example 13.
FIG. 29 shows the results of nanoflow cytometry detection of strepII-flag-PLXΔ-EV in Example 14.
FIG. 30 shows the results of nanoflow cytometry detection of FITC-streptactin labeled strepII-flag-PLXΔ-EV in Example 14.

### DETAILED DESCRIPTION

Unless otherwise defined, all technical and scientific terms used herein have the same meaning as understood by those of ordinary skill in the art.

Notwithstanding the numerical ranges and approximations of parameters shown in the broad scope of the present invention, the numerical values shown in the specific examples are set forth as accurately as possible. Any numerical values, however, are inherently bound to contain certain errors resulting from the standard deviation present in their respective measurements. Additionally, all ranges disclosed herein are to be understood to encompass any and all subranges subsumed therein. For example, a range described as "1 to 10" shall be deemed to include any and all subranges between the minimum value of 1 and the maximum value of 10 (including endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" shall be understood to be incorporated in its entirety.

It should also be noted that, as used in the present specification, the singular includes the plural form of its referent unless it is clearly and unambiguously limited to one referent.

The terms such as "comprise", "contain", and "include" as used herein are not intended to be limiting. In addition, "or" means "and/or" unless stated otherwise.

The patents, patent applications, cited publications, or descriptions cited herein are incorporated by reference in their entireties.

### Examples

Some preferred embodiments and aspects of the present invention will be further elaborated below in conjunction with specific examples. These examples should not be construed as limiting the scope of the present invention.

### Example 1: Materials and methods

The materials used in the examples of the present invention and their sources are shown in Table 1.

**Table 1**

| Reagent name | Manufacturer | Item number |
|---|---|---|
| KOP293-Ex | Kairui Biotech | K03256 |
| KT feed | Kairui Biotech | K40001 |
| D-PBS | Solarbio | D1040 |
| Trypan blue | Gibco | 15250-061 |
| Bradford kit | thermo | 23227 |
| PEI | Polyscince | 24765-1 |
| Anti-clumping | Gibco | 0010057AE |
| Flag antibody | Abcam | ab205606 |
| eGFP antibody | Abcam | ab290 |
| FBS | Gibco | 2110875CP |
| DMEM | Corning-Cellgro | 10-013-CV |
| PBS | Solarbio | P1020-500 |
| PS | Gibco | 15140-122 |
| L-Glutamine | Gibco | 25030149 |
| β-Mercaptoethanol | Solarbio | M8210 |
| 100kD Ultrafiltration tube | Millipore | UFC910096 |
| 96-well cell culture plates | Falcon | 353072 |
| Mouse Anti-CD3 | Invitrogen | 16-0031-86 |
| RPMI 1640 medium | Gibco | 11875093 |
| Recombinant mouse IL-12 (linked heterodimer) Protein | R&D | 10051-ML-010/CF |
| Mouse IFN-γPrecoated ELISA Kit | Dakewe | 1210002 |

### Experimental methods

### 1. Cell transfection

### 1) Cell preparation

On the day of transfection, the Expi293 F cells cultured in suspension were counted. The cell density was controlled at 2-5×10⁶/ml, and the viability was >95%. The cells were diluted to 2×10⁶/ml with a prewarmed medium, and the final volume was 30 ml.

### 2) Transfection

Preparation of solution A: 30 µg of plasmid was added by a pipette into a 15 ml centrifuge tube containing 1ml of Opti-MEM, and the mixture was mixed gently by blowing, and left to stand for 5 minutes.

Preparation of solution B: 60 µl of PEI (working solution concentration 1 µg/µl) was added by a pipette into a 15 ml centrifuge tube containing 1 ml of Opti-MEM, and the mixture was mixed gently by blowing, and left to stand for 5 minutes.

Solution B was added to solution A by a 1 ml pipette, and the mixture was mixed gently, and left to stand at room temperature for 20 minutes.

The mixed transfection reagent was added dropwise to Expi293 F cells while shaking gently.

### 3) Liquid addition after transfection

4-6 hours after transfection, the cell culture bottles were sterilized with 75% alcohol and transferred to a biological safety cabinet.

30 µl of Anti-clumping was added by a 100 µl pipette to a shake flask.

The cells were cultured in suspension in an incubator set to 37°C, 5% or 8% CO₂, 110 rpm.

### 4) Addition of KT Feed

22-24h after transfection, the cell culture bottles were sterilized with 75% ethanol and transferred to a biological safety cabinet. 0.6 ml of KT feed and 2 g/L glucose were added to the cell culture.

The incubator was set to 37°C, 5% or 8% CO₂ and 110 rpm according to the "Standard Operating Procedures for the Use, Cleaning and Maintenance of the 00-ZCZY-AS8V Shaking Incubator" (SOP-EQ-082).

### 5) Cell harvesting

Cells were harvested after 4 days of culture.

The cell suspension was transferred to a 50 ml sterile centrifuge tube, and centrifuged at 800 g and 4°C for 10 minutes. Then the cells and supernatant were separated.

The cell supernatant was transferred to a new 50 ml sterile centrifuge tube, and centrifuged at 4000 g and 4°C for 10 minutes.

The supernatant was collected, filtered and sterilized using a 0.22 µm filter, and transferred to a sterile 50 ml centrifuge tube for later use.

### 2. EV extraction

The cell supernatant was added to an ultracentrifuge tube, centrifuged at 16,000 × g for 2 h at 4°C, and then the supernatant was collected.

The supernatant obtained in the previous step was transferred to a clean ultracentrifuge tube and centrifuged at 100,000×g for 2 h at 4°C, and the supernatant was discarded.

After the centrifugation was completed, the EVs pellet was resuspended in PBS.

The resuspended EVs were added to a 100 kd ultrafiltration tube, and centrifuged at 4000×g for 10 min. Then the EVs were collected into a 1.5 mL centrifuge tube, and a total protein concentration was detected by BCA detection.

### 3. Western blotting (WB)

EVs of 10 µg total protein or cell lysate of 20 µg total protein, was added with an appropriate amount of 5× loading buffer to a final concentration of 1× loading buffer, and incubated at 95°C for 10 minutes. A running buffer (1L) was prepared according to the following formula: 100 mL of 10× running buffer (Cowin Biotech) + 900 mL ddH₂O. The running buffer was mixed gently and left for later use. A premade gel (Beyotime) of an appropriate concentration was installed in a gel running tank. After confirming that the assembled gel running tank did not leak, the prepared running buffer was added into the gel running tank so that the running buffer exceeded the gel surface. An appropriate amount of thermally denatured sample was carefully added by a pipette to each gel hole of the SDS-PAGE gel (be careful not to use excessive force when loading the sample to avoid the sample in one well contaminating another sample well). After correctly connecting the power supply of the gel running tank, the gel electrophoresis was performed at 80 V voltage. After the sample entered the separating gel, the gel electrophoresis was performed at a voltage of 120 V. After the target protein was separated, the gel electrophoresis was stopped.

An NC membrane of appropriate size was cut, soaked in methanol for at least 20 seconds, then washed with an appropriate amount of pre-cooled trans buffer, and left for later use. Preparation of trans buffer according to the following formula: 100 mL of 10x trans buffer (Cowin Biotech) + 200 mL of methanol + 700 mL of ddH₂O. Pre-cool after preparation. After the gel electrophoresis was completed, the gel was taken out and soaked in the pre-cooled trans buffer. A sandwich model was assembled according to the following order: black board - sponge - filter paper - gel - NC membrane - filter paper - sponge -whiteboard. Then the sandwich model was put into a membrane transfer tank in the order of "black board facing black board, white board facing white board". Two ice boxes frozen overnight at -80°C were put into the transfer tank, the transfer buffer was added into the entire sandwich model, the transfer tank was installed well, and the membrane transfer was performed at a constant current of 350 mA for 1 hour. Blocking: 5% BSA blocking buffer (dissolved in 1× TBST) was prepared. The membrane after transfer was taken out, placed in the blocking buffer, and blocked on a horizontal shaker at room temperature for 1 to 2 hours. Primary antibody incubation: After the blocking was completed, the membrane was washed 3 times with 1× TBST. A primary antibody solution was prepared in TBST according to desired proportion, and the membrane was incubated with the primary antibody solution for appropriate time. The preparation ratio and incubation time were recorded in Table 1. Secondary antibody incubation: after the incubation was completed, the membrane was washed 3 times with 1× TBST. A secondary antibody solution was prepared in TBST according to desired proportion, and the membrane was incubated with the second antibody solution for an appropriate time. ECL chemiluminescence color development: after the incubation with secondary antibody was completed, the membrane was washed 3 times with 1× TBST. Before the color development, the ECL chemiluminescence solution A and solution B were mixed at a ratio of 1:1, and then the membrane was developed on the Tanon-5200Multi, a chemiluminescence gel imaging system.

### 4. Quantitative detection of target protein

EVs of 1.2 µg total protein were added into 250 µl of RIPA lysis buffer (containing 100× protease inhibitor), and the mixture was mixed thoroughly, lysed at 4°C for 1 hour, and centrifuged at low speed to remove insoluble matter. 30 minutes before performing the ELISA experiment, the ELISA kit was taken out and left to stand at room temperature for equilibrium. Following the instructions, standards of different concentrations were prepared, and the samples were diluted with the diluent provided with the kit. The samples were incubated at 37°C for 90 min and then washed 4 times with a plate washer. 100 µl of antibody was added and incubated at 37°C for 60 minutes. The plate was washed 4 times with a plate washer. 100 µl of enzyme conjugate was added, and incubated at 37°C for 30 minutes. After the plate was washed 4 times with the plate washer, color was developed for 15 minutes. Then the plate was added with stopping solution, and read using a microplate reader.

### 5. Measurement of EV concentration

The concentration and particle size of EVs were measured using Flow NanoAnalyzer N30E (NanoFCM Inc., Xiamen, China) equipped with 488nm wavelength laser. First, calibration of quality control microspheres (250 nm SiNPs) and identification of mixed microspheres (68-155 S16-Exo) were performed with the instrument. Then EVs were diluted with PBS to a concentration range of 1×10⁸-6×10⁸particles/mL (the detected total particle number was 2000-12000particles/min). Sample data was collected according to the collection time of 60s, and the concentration and particle size distribution of EVs were analyzed using Flow NanoAnalyzer software (NF Profession version 2.0).

### 6. IL-12 activity assay in vitro

70 µm cell strainer was put into a culture dish, and added with 3 mL of mouse lymphocyte separation solution.

The spleen of the C57BL/6 mouse was taken under aseptic condition, placed in the culture dish prepared in the previous step, and ground by gently pushing the rubber end of a push rod of a 5 mL syringe until there is no red clump.

After the cell strainer was washed with 1mL of mouse lymphocyte separation buffer, the cell suspension was collected into a 15mL centrifuge tube.

1mL of RPMI-1640 was slowly added to the above-mentioned 15mL centrifuge tube against the wall of the centrifuge tube. After the acceleration and deceleration of the centrifuge was set to the third gear, the centrifuge tube was centrifuged at 800g for 30 minutes.

The supernatant was discarded. The cells were resuspended in 10mL RPMI 1640, centrifuged at 250g for 10 minutes, collected and counted. The cell density was adjusted to 2×10⁶ cells/mL.

Mouse CD3 antibody (0.5µg/mL) was added and mixed evenly. The cells were placed at 37°C, 5% CO₂, and cultured for 2 days. During this period, the cell status (agglomeration) was observed.

On the third day, the stimulated mouse spleen cells were collected and centrifuged at 300g for 5 minutes.

The supernatant was discarded, and the cells were resuspended in mouse spleen cell culture medium, and centrifuged at 300g for 5 minutes.

The supernatant was discarded, and the cells were resuspended in mouse spleen cell culture medium, and counted (cell viability >80%).

According to the plate layout, cells were transferred into a 96-well plate at 1.5x10⁵/90µL per well and placed in a cell culture incubator at 37°C and 5% CO₂ for later use.

According to the plate layout, 10 µL/well of the sample diluted in gradient was added to the 96-well plate with cells, and mixed gently. 200 µL of sterile water was added to the surrounding wells to prevent evaporation. The cells were placed at 37°C, 5% CO₂, and cultured for 40 hours.

The next day, the plate with the incubated spleen cells and samples was centrifuged at 1500 rpm for 5 minutes. 60 µL of the supernatant was collected, and Mouse-IFNγ was detected with an ELISA KIT.

### Example 2: Expression and enrichment of plexin family members in EVs

The inventor accidentally found that members of the plexin family are highly expressed in EVs, and have potential prospect for carrying and enriching target proteins on EVs. PLXNA1 eukaryotic expression plasmid was constructed and PLXNA1 protein was overexpressed in cells. EVs were collected according to the method described in Example 1, and PLXNA1 expression in EV-free cell lysates and in EVs was detected by WB. The results showed that PLXNA1 protein was sorted to EVs and has a high expression level, indicating its enrichment in EVs.

The full-length sequence of PLXNA1 used in the experiment is as follows:

































 (SEQ ID NO: 1).

### Example 3: Construction and expression of truncated PLXNA1

PLXNA1 has an extracellular region, a transmembrane region, and an intracellular region. Both the extracellular and intracellular regions are involved in its biological functions, so full-length PLXNA1 is not suitable as a fusion partner to carry the target protein. It was unexpectedly found that PLXNA1 with part of the extracellular region and/or intracellular region deleted can still carry the target protein to EVs. A series of truncated PLXNA1 were constructed, and two different polypeptides, mIL12 and eGFP (as markers) were fused to the N-terminal and C-terminal ends of the truncated PLXNA1 respectively. See FIG. 2 for a schematic structural diagram. In addition, the fusion protein also carried a Flag tag.

The amino acid sequences comprised in the plasmid constructed in this example (P in the plasmid name is the abbreviation of plasmid) are as follows:
P292 (863-1316): Truncated PLXNA1, comprising four IPT domains-transmembrane region-part of the adjacent sequence 
DPKILKLSPETGPRQGGTRLTITGENLGLRFEDVRLGVRVGKVLCSPVESEYISAE
QIVCEIGDASSVRAHDALVEVCVRDCSPHYRALSPKRFTFVTPTFYRVSPSRGPLSGGTWI
GIEGSHLNAGSDVAVSVGGRPCSFSWRNSREIRCLTPPGQSPGSAPIIININRAQLTNPEVKY
NYTEDPTILRIDPEWSINSGGTLLTVTGTNLATVREPRIRAKYGGIERENGCLVYNDTTMV
CRAPSVANPVRSPPELGERPDELGFVMDNVRSLLVLNSTSFLYYPDPVLEPLSPTGLLELK
PSSPLILKGRNLLPPAPGNSRLNYTVLIGSTPCTLTVSETQLLCEAPNLTGQHKVTVRAGGF
EFSPGTLQVYSDSLLTLPAIVGIGGGGGLLLLVIVAVLIAYKRKSRDADRTLKRLQLQMDN
LESRVALECKEAFAELQTDIHELTNDLDG (SEQ ID NO: 2)
P235 (863-1300): Truncated PLXNA1, comprising four IPT domains-transmembrane region 
DPKILKLSPETGPRQGGTRLTITGENLGLRFEDVRLGVRVGKVLCSPVESEYISAE
QIVCEIGDASSVRAHDALVEVCVRDCSPHYRALSPKRFTFVTPTFYRVSPSRGPLSGGTWI
GIEGSHLNAGSDVAVSVGGRPCSFSWRNSREIRCLTPPGQSPGSAPIIININRAQLTNPEVKY
NYTEDPTILRIDPEWSINSGGTLLTVTGTNLATVREPRIRAKYGGIERENGCLVYNDTTMV
CRAPSVANPVRSPPELGERPDELGFVMDNVRSLLVLNSTSFLYYPDPVLEPLSPTGLLELK
PSSPLILKGRNLLPPAPGNSRLNYTVLIGSTPCTLTVSETQLLCEAPNLTGQHKVTVRAGGF
EFSPGTLQVYSDSLLTLPAIVGIGGGGGLLLLVIVAVLIAYKRKSRDADRTLKRLQLQMDN
LESRVALECKEAF (SEQ ID NO: 3)
P236 (960-1300): Truncated PLXNA1, comprising three IPT domains-transmembrane region 
TPTFYRVSPSRGPLSGGTWIGIEGSHLNAGSDVAVSVGGRPCSFSWRNSREIRCLT
PPGQSPGSAPIIININRAQLTNPEVKYNYTEDPTILRIDPEWSINSGGTLLTVTGTNLATVRE
PRIRAKYGGIERENGCLVYNDTTMVCRAPSVANPVRSPPELGERPDELGFVMDNVRSLLV
LNSTSFLYYPDPVLEPLSPTGLLELKPSSPLILKGRNLLPPAPGNSRLNYTVLIGSTPCTLTV
SETQLLCEAPNLTGQHKVTVRAGGFEFSPGTLQVYSDSLLTLPAIVGIGGGGGLLLLVIVA
VLIAYKRKSRDADRTLKRLQLQMDNLESRVALECKEAF (SEQ ID NO: 4)
P237 (1046-1300): Truncated PLXNA1, comprising two IPT domains-transmembrane region 
EDPTILRIDPEWSINSGGTLLTVTGTNLATVREPRIRAKYGGIERENGCLVYNDTT
MVCRAPSVANPVRSPPELGERPDELGFVMDNVRSLLVLNSTSFLYYPDPVLEPLSPTGLLE
LKPSSPLILKGRNLLPPAPGNSRLNYTVLIGSTPCTLTVSETQLLCEAPNLTGQHKVTVRA
GGFEFSPGTLQVYSDSLLTLPAIVGIGGGGGLLLLVIVAVLIAYKRKSRDADRTLKRLQLQ
MDNLESRVALECKEAF (SEQ ID NO: 5)
P238 (1143-1300): Truncated PLXNA1, comprising one IPT domain-transmembrane region

SFLYYPDPVLEPLSPTGLLELKPSSPLILKGRNLLPPAPGNSRLNYTVLIGSTPCTL
TVSETQLLCEAPNLTGQHKVTVRAGGFEFSPGTLQVYSDSLLTLPAIVGIGGGGGLLLLVI
VAVLIAYKRKSRDADRTLKRLQLQMDNLESRVALECKEAF (SEQ ID NO: 6)

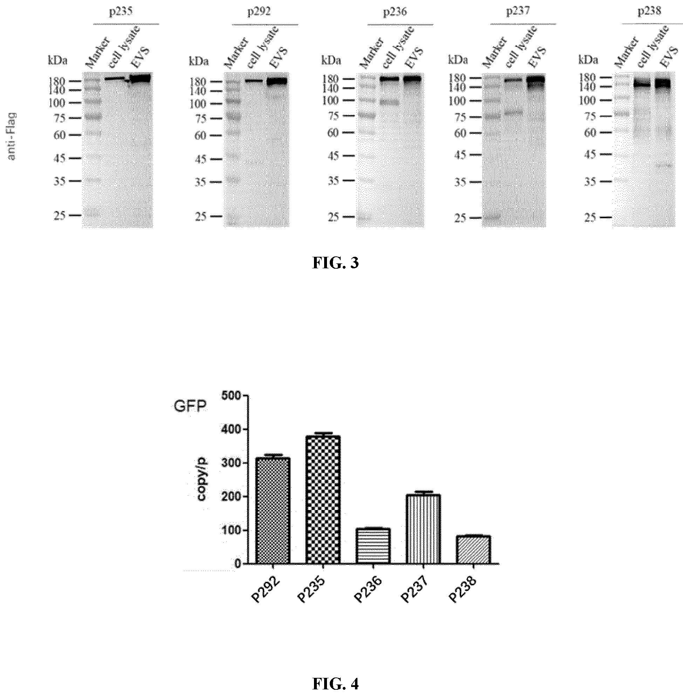

According to the experimental method described in Example 1, the above plasmids were constructed. Each of the plasmid was transfected into suspended 293 cells and cultured until the cells were harvested. When the supernatant was harvested, the supernatant was separated by low-speed centrifugation for later use. The cells obtained by centrifugation were lysed, and the total protein of the cell lysate was quantified using BCA. EVs were separated from the supernatant of the harvested cells by ultra-high-speed centrifugation, and the total protein of EVs was quantified using BCA. The cell lysates and EVs with the same total protein amount were analyzed using Western blotting, and the intensity of the bands was compared to determine whether the target proteins were enriched in EVs. The results in FIG. 3 show that different truncated PLXNA1 still have the ability of enriching target proteins to EVs.

### Detection of the number of eGFP on EVs by ELISA

1) The particle concentration *CONC_{particle}* (particles/mL) of the obtained EVs was detected using nanoflow cytometry.
2) eGFP concentration *CONC_{eGFP}* (ng/mL) was detected using ELISA kit.
3) The average copy number of eGFP on a single EV particle was calculated according to the formula *copy*/*p=(CONC_{eGFP}*N_{A})*/*(CONC_{particle}*M_{eGFP}),* where *N_{A}* represents Avogadro's constant (about 6.02× 10²³), and *M_{eGFP}* represents the molar mass of eGFP.

The results are shown in FIG. 4. It shows that P292 and P235 have high copy numbers on single EV particles. Based on this result, the subsequent examples used truncated P235, namely PLXNA1 (863-1300), as the fusion partner for constructing fusion proteins.

### Example 4: Comparison with PTGFRN and Lamp2b

PTGFRN and Lamp2b are EV biomarker proteins reported in the art and can carry target proteins to EVs (Delivery of siRNA to the mouse brain by systemic injection of targeted exosomes, Nature Biotechnology volume 29, pages341-345 (2011)). The same vector was used to construct expression plasmids for PLXNA1, PTGFRN and Lamp2b fusion proteins.

P235: mIL12-PLXNA1(863-1300)-Flag-eGFP.

P320: mIL12-PTGFRN(FL)-Flag-eGFP; the fusion protein sequence is as follows:

MCHQQLVISWFSLVFLASPLVAMWELEKDVYVVEVDWTPDAPGETVNLTCDTP
EEDDITWTSDQRHGVIGSGKTLTITVKEFLDAGQYTCHKGGETLSHSHLLLHKKENGIWS
TEILKNFKNKTFLKCEAPNYSGRFTCSWLVQRNMDLKFNIKSSSSSPDSRAVTCGMASLS
AEKVTLDQRDYEKYSVSCQEDVTCPTAEETLPIELALEARQQNKYENYSTSFFIRDIIKPD
PPKNLQMKPLKNSQVEVSWEYPDSWSTPHSYFSLKFFVRIQRKKEKMKETEEGCNQKG
AFLVEKTSTEVQCKGGNVCVQAQDRYYNSSCSKWACVPCRVRSGGSGGGSGGGGSGGG
GSGGGSGGRVIPVSGPARCLSQSRNLLKTTDDMVKTAREKLKHYSCTAEDIDHEDITRDQ
TSTLKTCLPLELHKNESCLATRETSSTTRGSCLPPQKTSLMMTLCLGSIYEDLKMYQTEFQ

AINAALQNHNHQQIILDKGMLVAIDELMQSLNHNGETLRQKPPVGEADPYRVKMKLCIL
LHAFSTRVVTINRVMGYLSSAGGGGSGGGGSRVVRVPTATLVRVVGTELVIPCNVSDYDG
PSEQNFDWSFSSLGSSFVELASTWEVGFPAQLYQERLQRGEILLRRTANDAVELHIKNVQP
SDQGHYKCSTPSTDATVQGNYEDTVQVKVLADSLHVGPSARPPPSLSLREGEPFELRCTA
ASASPLHTHLALLWEVHRGPARRSVLALTHEGRFHPGLGYEQRYHSGDVRLDTVGSDAY
RLSVSRALSADQGSYRCIVSEWIAEQGNWQEIQEKAVEVATVVIQPSVLRAAVPKNVSVA
EGKELDLTCNITTDRADDVRPEVTWSFSRMPDSTLPGSRVLARLDRDSLVHSSPHVALSH
VDARSYHLLVRDVSKENSGYYYCHVSLWAPGHNRSWHKVAEAVSSPAGVGVTWLEPDY
QVYLNASKVPGFADDPTELACRVVDTKSGEANVRFTVSWYYRMNRRSDNVVTSELLAV
MDGDWTLKYGERSKQRAQDGDFIFSKEHTDTFNFRIQRTTEEDRGNYYCVVSAWTKQR
NNSWVKSKDVFSKPVNIFWALEDSVLVVKARQPKPFFAAGNTFEMTCKVSSKNIKSPRYS
VLIMAEKPVGDLSSPNETKYIISLDQDSVVKLENWTDASRVDGVVLEKVQEDEFRYRMY
QTQVSDAGLYRCMVTAWSPVRGSLWREAATSLSNPIEIDFQTSGPIFNASVHSDTPSVIRG
DLIKLFCIITVEGAALDPDDMAFDVSWFAVHSFGLDKAPVLLSSLDRKGIVTTSRRDWKS
DLSLERVSVLEFLLQVHGSEDQDFGNYYCSVTPWVKSPTGSWQKEAEIHSKPVFITVKM
DVLNAFKYPLLIGVGLSTVIGLLSCLIGYCSSHWCCKKEVQETRRERRRLMSMEMDGGG
GSDYKDHDGDYKDHDIDYKDDDDKGGGGSGGGGSVSKGEELFTGVVPILVELDGDVN
GHKFSVSGEGEGDATYGKLTLKFICTTGKLPVPWPTLVTTLTYGVQCFSRYPDHMKQHD
FFKSAMPEGYVQERTIFFKDDGNYKTRAEVKFEGDTLVNRIELKGIDFKEDGNILGHKLE
YNYNSHNVYIMADKQKNGIKVNFKIRHNIEDGSVQLADHYQQNTPIGDGPVLLPDNHYL
STQSALSKDPNEKRDHMVLLEFVTAAGITLGMDELYK (SEQ ID NO: 7)

P321: mIL12-Lamp2b(FL)-Flag-eGFP; the fusion protein sequence is as follows:

MCHQQLVISWFSLVFLASPLVAMWELEKDVYVVEVDWTPDAPGETVNLTCDTP
EEDDITWTSDQRHGVIGSGKTLTITVKEFLDAGQYTCHKGGETLSHSHLLLHKKENGIWS
TEILKNFKNKTFLKCEAPNYSGRFTCSWLVQRNMDLKFNIKSSSSSPDSRAVTCGMASLS
AEKVTLDQRDYEKYSVSCQEDVTCPTAEETLPIELALEARQQNKYENYSTSFFIRDIIKPD
PPKNLQMKPLKNSQVEVSWEYPDSWSTPHSYFSLKFFVRIQRKKEKMKETEEGCNQKG
AFLVEKTSTEVQCKGGNVCVQAQDRYYNSSCSKWACVPCRVRSGGSGGGSGGGGSGGG
GSGGGSGGRVIPVSGPARCLSQSRNLLKTTDDMVKTAREKLKHYSCTAEDIDHEDITRDQ
TSTLKTCLPLELHKNESCLATRETSSTTRGSCLPPQKTSLMMTLCLGSIYEDLKMYQTEFQ

AINAALQNHNHQQIILDKGMLVAIDELMQSLNHNGETLRQKPPVGEADPYRVKMKLCIL
LHAFSTRVVTINRVMGYLSSAGGGGSGGGGSLELNLTDSENATCLYAKWQMNFTVRYET
TNKTYKTVTISDHGTVTYNGSICGDDQNGPKIAVQFGPGFSWIANFTKAASTYSIDSVSFS
YNTGDNTTFPDAEDKGILTVDELLAIRIPLNDLFRCNSLSTLEKNDVVQHYWDVLVQAFV
QNGTVSTNEFLCDKDKTSTVAPTIHTTVPSPTTTPTPKEKPEAGTYSVNNGNDTCLLATM
GLQLNITQDKVASVININPNTTHSTGSCRSHTALLRLNSSTIKYLDFVFAVKNENRFYLKE
VNISMYLVNGSVFSIANNNLSYWDAPLGSSYMCNKEQTVSVSGAFQINTFDLRVQPFNV
TQGKYSTAQECSLDDDTILIPIIVGAGLSGLIIVIVIAYVIGRRKSYAGYQTLGGGGSDYKD
HDGDYKDHDIDYKDDDDKGGGGSGGGGSVSKGEELFTGVVPILVELDGDVNGHKFSVS
GEGEGDATYGKLTLKFICTTGKLPVPWPTLVTTLTYGVQCFSRYPDHMKQHDFFKSAMP
EGYVQERTIFFKDDGNYKTRAEVKFEGDTLVNRIELKGIDFKEDGNILGHKLEYNYNSHN
VYIMADKQKNGIKVNFKIRHNIEDGSVQLADHYQQNTPIGDGPVLLPDNHYLSTQSALS
KDPNEKRDHMVLLEFVTAAGITLGMDELYK (SEQ ID NO: 8)

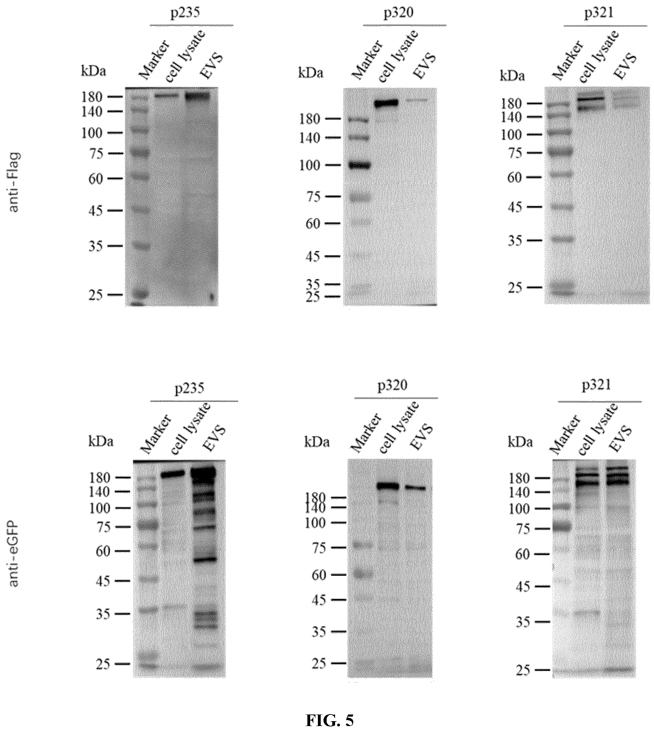

The three plasmids were transfected into HEK293 respectively, and cultured. The cell supernatants were collected, and EVs were extracted. The cell lysate, the supernatant and the EVs were loaded at the same amount of proteins, and the expression of the fusion protein (anti-Flag antibody and anti-eGFP antibody were used) was detected by WB. The specific experimental operations are the same as in Example 3, and the results are shown in FIG. 5.

As shown in FIG. 5, compared with the reported EV proteins PTGFRN and Lamp2b, the truncated PLXNA1 fusion protein has a higher enrichment level in EVs.

### Example 5: Other proteins of plexin family

In order to verify whether other members in the same family also have similar functions, other members PLXNA2, PLXNB1, PLXNB2, PLXNC1 and PLXND1 were selected to construct plasmids.
P240: mIL12-PLXNB2(802-1217)-Flag-eGFP
P241: mIL12-PLXNA2(857-1306)-Flag-eGFP
P242: mIL12-PLXNB1(1068-1558)-Flag-eGFP
P433: mIL12-PLXNC1(661-1003)-Flag-eGFP
P434: mIL12-PLXNDI(886-1341)-Flag-eGFP
PLXNB2 (802-1217) amino acid sequence: 
PPVITRIQPETGPLGGGIRITILGSNLGVQAGDIQRISVAGRNCSFQPERYSVSTRIV
CVIEAAETPFTGGVEVDVFGKLGRSPPNVQFTFQQPKPLSVEPQQGPQAGGTTLTIHGTH
LDTGSQEDVRVTLNGVPCKVTKFGAQLQCVTGPQATRGQMLLEVSYGGSPVPNPGIFFT
YRENPVLRAFEPLRSFASGGRSINVTGQGFSLIQRFAMVVIAEPLQSWQPPREAESLQPMT
VVGTDYVFHNDTKVVFLSPAVPEEPEAYNLTVLIEMDGHRALLRTEAGAFEYVPDPTFEN
FTGGVKKQVNKLIHARGTNLNKAMTLQEAEAFVGAERCTMKTLTETDLYCEPPEVQPPP
KRRQKRDTTHNLPEFIVKFGSREWVLGRVEYDTRVSDVPLSLILPLVIVPMVVVIAVSVYC
YWRKSQQAEREYEKIKSQLEGLEESVRDRCKKEFTDLMIEMEDQTNDV (SEQ ID NO: 9)
PLXNA2 (857-1306) amino acid sequence: 
NPQITEILTVSGPPEGGTRVTIHGVNLGLDFSEIAHHVQVAGVPCTPLPGEYIIAEQ
IVCEMGHALVGTTSGPVRLCIGECKPEFMTKSHQQYTFVNPSVLSLNPIRGPESGGTMVTI
TGHYLGAGSSVAVYLGNQTCEFYGRSMSEIVCVSPPSSNGLGPVPVSVSVDRAHVDSNL
QFEYIDDPRVQRIEPEWSIASGHTPLTITGFNLDVIQEPRIRVKFNGKESVNVCKVVNTTTL
TCLAPSLTTDYRPGLDTVERPDEFGFVFNNVQSLLIYNDTKFIYYPNPTFELLSPTGVLDQ
KPGSPIILKGKNLCPPASGGAKLNYTVLIGETPCAVTVSETQLLCEPPNLTGQHKVMVHV
GGMVFSPGSVSVISDSLLTLPAIVSIAAGGSLLLIIVIIVLIAYKRKSRENDLTLKRLQMQMD
NLESRVALECKEAFAELQTDINELTSD (SEQ ID NO: 10)
PLXNB1 (1068-1558) amino acid sequence: 
PAPLIHSVEPLTGPVDGGTRVTIRGSNLGQHVQDVLGMVTVAGVPCAVDAQEYE
VSSSLVCITGASGEEVAGATAVEVPGRGRGVSEHDFAYQDPKVHSIFPARGPRAGGTRLTL
NGSKLLTGRLEDIRVVVGDQPCHLLPEQQSEQLRCETSPRPTPATLPVAVWFGATERRLQR
GQFKYTLDPNITSAGPTKSFLSGGREICVRGQNLDVVQTPRIRVTVVSRMLQPSQGLGRR
RRVVPETACSLGPSCSSQQFEEPCHVNSSQLITCRTPALPGLPEDPWVRVEFILDNLVFDFA
TLNPTPFSYEADPTLQPLNPEDPTMPFRHKPGSVFSVEGENLDLAMSKEEVVAMIGDGPC
VVKTLTRHHLYCEPPVEQPLPRHHALREAPDSLPEFTVQMGNLRFSLGHVQYDGESPGA
FPVAAQVGLGVGTSLLALGVIIIVLMYRRKSKQALRDYKKVQIQLENLESSVRDRCKKEF
 TDLMTEMTDLTSDL (SEQ ID NO: 11)
PLXNC1 (661-1003) amino acid sequence: 
VFYIKSIEPQKVSTLGKSNVIVTGANFTRASNITMILKGTSTCDKDVIQVSHVLN
DTHMKFSLPSSRKEMKDVCIQFDGGNCSSVGSLSYIALPHCSLIFPATTWISGGQNITMMG
RNFDVIDNLIISHELKGNINVSEYCVATYCGFLAPSLKSSKVRTNVTVKLRVQDTYLDCGT
LQYREDPRFTGYRVESEVDTELEVKIQKENDNFNISKKDIEITLFHGENGQLNCSFENITR
NQDLTTILCKIKGIKTASTIANSSKKVRVKLGNLELYVEQESVPSTWYFLIVLPVLLVIVIFA
AVGVTRHKSKELSRKQSQQLELLESELRKEIRDGFAELQMDK (SEQ ID NO: 12)
PLXND1 (886-1341) amino acid sequence:

GTCPAPEIHAIEPLSGPLDGGTLLTIRGRNLGRRLSDVAHGVWIGGVACEPLPDR
YTVSEEIVCVTGPAPGPLSGVVTVNASKEGKSRDRFSYVLPLVHSLEPTMGPKAGGTRITI
HGNDLHVGSELQVLVNDTDPCTELMRTDTSIACTMPEGALPAPVPVCVRFERRGCVHGN
LTFWYMQNPVITAISPRRSPVSGGRTITVAGERFHMVQNVSMAVHHIGREPTLCKVLNST
LITCPSPGALSNASAPVDFFINGRAYADEVAVAEELLDPEEAQRGSRFRLDYLPNPQFSTAK
REKWIKHHPGEPLTLVIHKEQDSLGLQSHEYRVKIGQVSCDIQIVSDRIIHCSVNESLGAAV
GQLPITIQVGNFNQTIATLQLGGSETAIIVSIVICSVLLLLSVVALFVFCTKSRRAERYWQKT
LLQMEEMESQIREEIRKGFAELQTDMTDLTKELN (SEQ ID NO: 13)

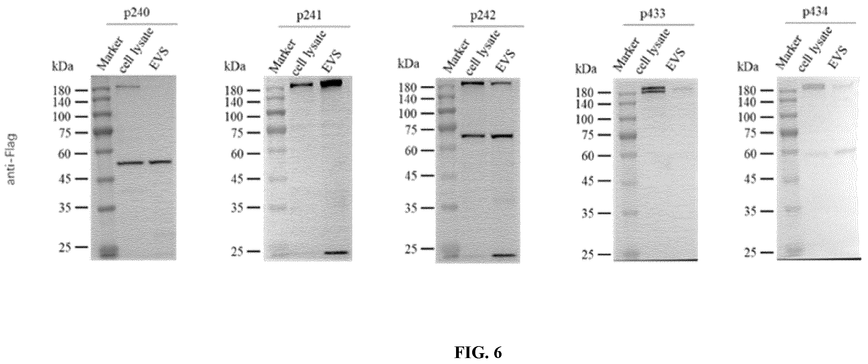

The expression of fusion protein, EV collection and WB detection were the same as described in Example 3. The WB result of anti-Flag is shown in FIG. 6. Experimental results show that the above five fusion proteins were all expressed on EVs, among which PLXNA2 was significantly enriched on EVs.

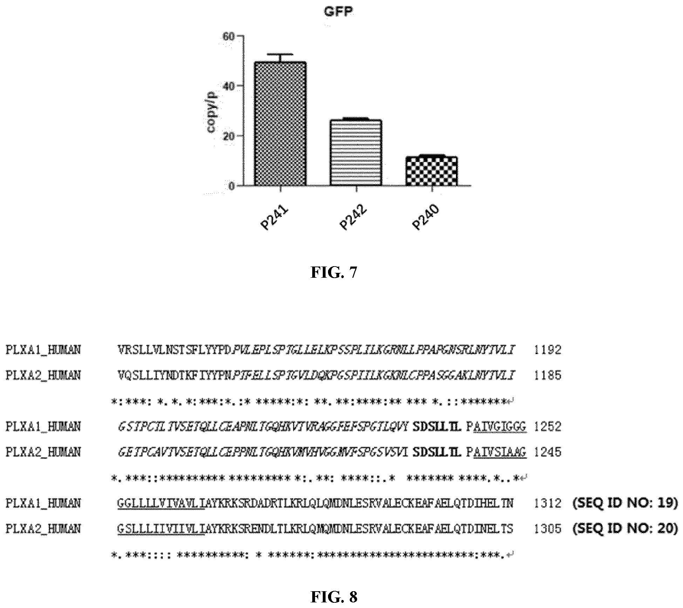

The particle concentration and eGPF content of three samples of P240, P241 and P242 were detected to calculate the copy number of eGFP on each EV. The results are shown in FIG. 7. It can be seen that the EVs of the three samples P240, P241 and P242 all carry the corresponding fusion proteins at different amounts.

### Example 6: Identification of polypeptide sequences critical for EV enrichment

Example 3 shows that the truncated PLXNA1 still has an obvious function of enriching target proteins to EVs. Through sequence alignment, it was found that there is a relatively conserved 7-amino acid sequence SDSLLTL (SEQ ID NO: 14) between the transmembrane regions of PLXNA1 and the IPT domains, also the transmembrane regions of PLXNA2 and the IPT domains (see FIG. 8). It is speculated that this sequence may make a special contribution to the active sorting of EVs.

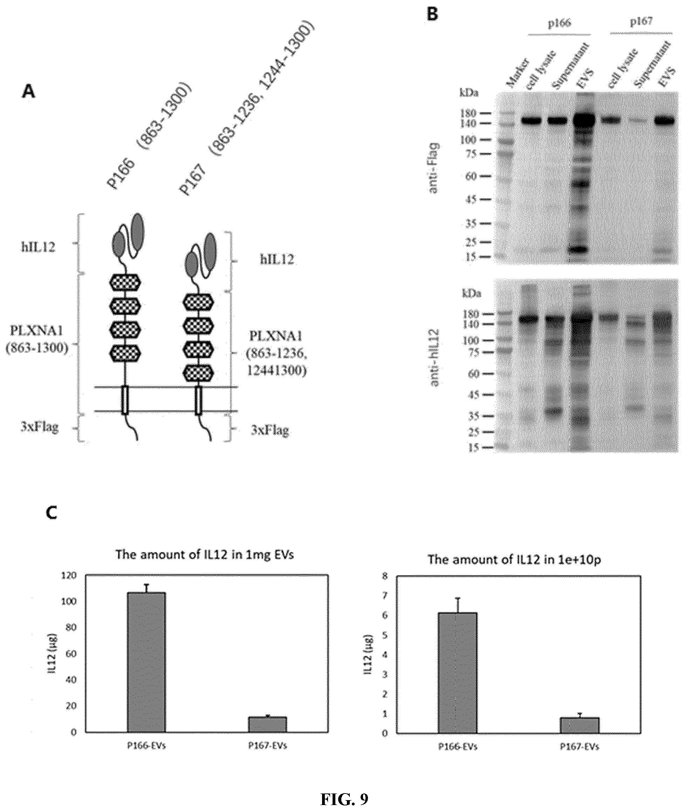

On this basis, plasmids P166 and P167 were constructed (FIG. 9A), in which P167 did not contain the conserved 8 amino acid sequence compared with P166. The WB results are shown in FIG. 9B. When the sequence SDSLLTL (SEQ ID NO: 14) (position 1237-1243 in the full length) was deleted, the expression levels of the fusion protein in the cell supernatant and EVs were significantly decreased.

In addition, the total IL12 in the supernatant and the IL12 in EVs were detected using ELISA. The results show that the proportion of IL12 in EVs in the supernatant of P166 sample was significantly higher than that of P167. This result demonstrates that the sequence of PLXNA1 (1237-1243) is a key sequence and plays an important role in the active sorting of fusion proteins onto EVs.

### Example 7: Impact of mutations in key sequence on protein sorting

In order to study how the amino acids at each position in the key sequence SDSLLTL (SEQ ID NO: 14) affect the sorting efficiency of the fusion protein, a mutation study was conducted on this sequence.

First, each amino acid in this sequence was mutated to alanine (A) one by one. The sequences are as follows:
ADSLLTL (SEQ ID NO: 21)
SASLLTL (SEQ ID NO: 22)
SDALLTL (SEQ ID NO: 23)
SDSALTL (SEQ ID NO: 24)
SDSLATL (SEQ ID NO: 25)
SDSLLAL (SEQ ID NO: 26)
SDSLLTA (SEQ ID NO: 27)

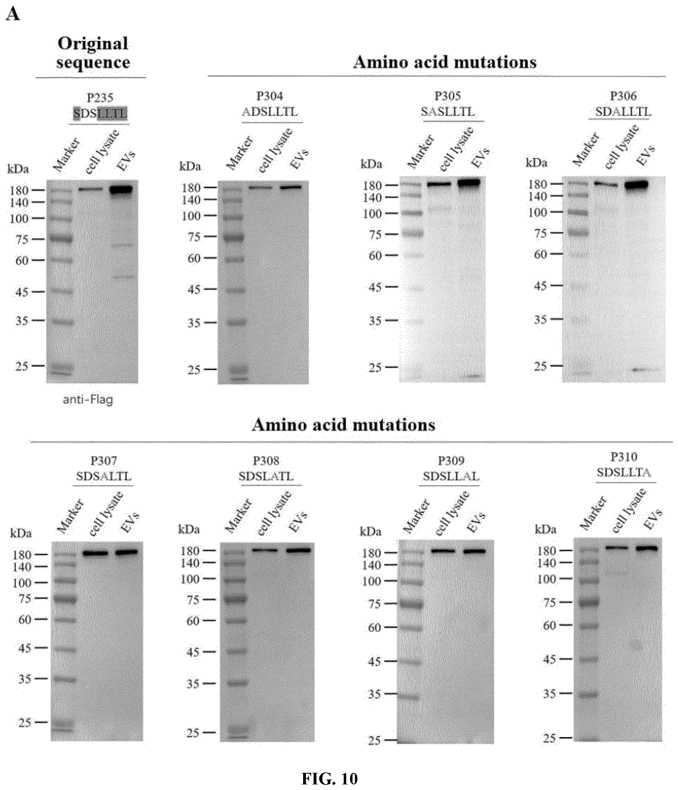

The constructed plasmid was then transiently transfected into HEK293 cells, the EVs in the supernatant were extracted, and EVs and cell lysates with equal total protein amounts were subjected to WB experiments (using anti-Flag antibody). The results show that P304 (S1237A), P307 (L1240A), P308 (L1241A), P309 (T1242A), and P310 (L1243A) mutations had a significant impact on the sorting behavior of fusion proteins to EVs, and the sorting efficiency was significantly reduced. While P305 (D1238A) and P306 (S1239A) did not bring significant changes (FIG. 10A).

In addition, similar amino acid mutations were also carried out at different positions in the key sequence. The sequences are as follows:
SESLLTL (SEQ ID NO: 28)
SDSLITL (SEQ ID NO: 29)

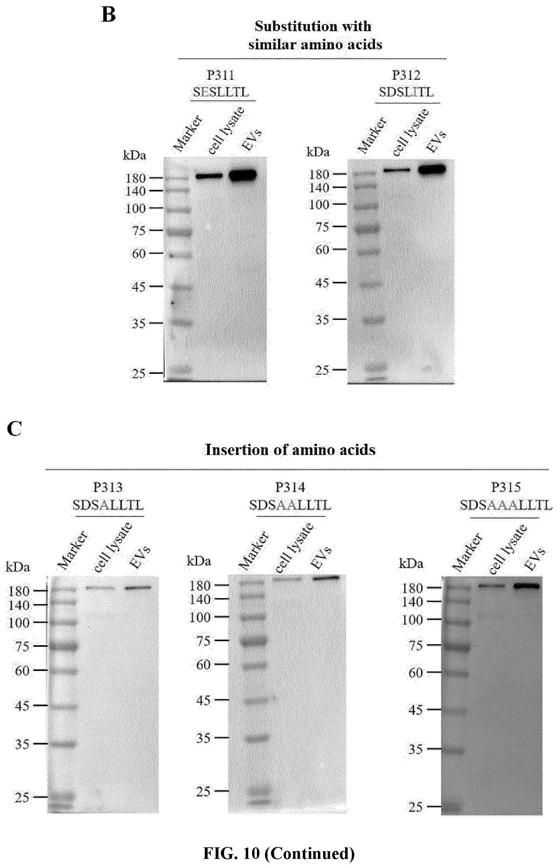

The results show that P311 (D1238E) and P312 (L1241I) also hardly changed the sorting efficiency of the fusion protein (FIG. 10B).

1, 2 or 3 alanine were inserted between amino acid S at position 1239 and amino acid L at position 1240. The sequences are as follows:
SDSALLTL (SEQ ID NO: 30)
SDSAALLTL (SEQ ID NO: 31)
SDSAAALLTL (SEQ ID NO: 32)

The results show that inserting amino acids of different lengths at this position changed the sorting efficiency of the fusion protein (FIG. 10C).

### Example 8: Truncated PLXNA1 sorts different target proteins to EVs

In order to verify when different proteins or polypeptides are fused, whether POI can be sorted to EVs, plasmids expressing different fusion proteins were constructed, and transiently transfected into HEK293 cells. The cell lysates and EVs were collected, and the target proteins were detected using WB. Among them, S-RBD represents the COVID-19 virus spike protein, eGFP represents green fluorescent protein, nanoluc represents a new luciferase with a size of about 171 amino acids, and mCD3 scFab represents the single-chain antigen-binding fragment of mouse CD3. The fusion protein sequence carried by the plasmid is as follows:
P322: S-RBD-PLXNA1 (863-1300)-Flag-eGFP, the fusion protein sequence is as follows: 
MCHQQLVISWFSLVFLASPLVARVQPTESIVRFPNITNLCPFGEVFNATRFASVYAWNRKRI
SNCVADYSVLYNSASFSTFKCYGVSPTKLNDLCFTNVYADSFVIRGDEVRQIAPGQTGKI
ADYNYKLPDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKKSTNLVKN
KCVNFGGGGSGGGGSDPKILKLSPETGPRQGGTRLTITGENLGLRFEDVRLGVRVGKVLC
SPVESEYISAEQIVCEIGDASSVRAHDALVEVCVRDCSPHYRALSPKRFTFVTPTFYRVSPS
RGPLSGGTWIGIEGSHLNAGSDVAVSVGGRPCSFSWRNSREIRCLTPPGQSPGSAPIIININR
AQLTNPEVKYNYTEDPTILRIDPEWSINSGGTLLTVTGTNLATVREPRIRAKYGGIERENG
CLVYNDTTMVCRAPSVANPVRSPPELGERPDELGFVMDNVRSLLVLNSTSFLYYPDPVLE
PLSPTGLLELKPSSPLILKGRNLLPPAPGNSRLNYTVLIGSTPCTLTVSETQLLCEAPNLTGQ
HKVTVRAGGFEFSPGTLQVYSDSLLTLPAIVGIGGGGGLLLLVIVAVLIAYKRKSRDADRT
LKRLQLQMDNLESRVALECKEAFGGGGSDYKDHDGDYKDHDIDYKDDDDKGGGGSGG
GGSVSKGEELFTGVVPILVELDGDVNGHKFSVSGEGEGDATYGKLTLKFICTTGKLPVPW
PTLVTTLTYGVQCFSRYPDHMKQHDFFKSAMPEGYVQERTIFFKDDGNYKTRAEVKFEG
DTLVNRIELKGIDFKEDGNILGHKLEYNYNSHNVYIMADKQKNGIKVNFKIRHNIEDGSV
QLADHYQQNTPIGDGPVLLPDNHYLSTQSALSKDPNEKRDHMVLLEFVTAAGITLGMDE
LYK (SEQ ID NO: 16)
P323: mCD3 scFab-PLXNA1(863-1300)-Flag-eGFP; the fusion protein sequence is as follows: 
MCHQQLVISWFSLVFLASPLVAMGRLASRPLLLALLSLALCRGRMKIICLALVALLLTAQP
AMAEIVLTQSPATLSLSPGERATLSCRASQSVSSYLAWYQQKPGQAPRLLIYDASNRATGI
PARFSGSGSGTDFTLTISSLEPEDFAVYYCQQRSNWPPLTFGGGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGECGGSSGSGSGSTGTSSSGTGTSAGTT
 
GTSASTSGSGSGGGGGSGGGGSAGGTATAGASSGSQVQLVESGGGVVQPGRSLRLSCAA
SGFKFSGYGMHWVRQAPGKGLEWVAVIWYDGSKKYYVDSVKGRFTISRDNSKNTLYLQ
MNSLRAEDTAVYYCARQMGYWHFDLWGRGTLVTVSSASTKGPSVFPLAPSSKSTSGGTA
ALGCLVKDYFPEPVTVSWNSGALTSGVHTFPAVLQSSGLYSLSSVVTVPSSSLGTQTYICN
VNHKPSNTKVDKKVEPKSCDKTHTGGGGSGGGGSDPKILKLSPETGPRQGGTRLTITGEN
LGLRFEDVRLGVRVGKVLCSPVESEYISAEQIVCEIGDASSVRAHDALVEVCVRDCSPHY
RALSPKRFTFVTPTFYRVSPSRGPLSGGTWIGIEGSHLNAGSDVAVSVGGRPCSFSWRNSR
EIRCLTPPGQSPGSAPIIININRAQLTNPEVKYNYTEDPTILRIDPEWSINSGGTLLTVTGTNL
ATVREPRIRAKYGGIERENGCLVYNDTTMVCRAPSVANPVRSPPELGERPDELGFVMDN
VRSLLVLNSTSFLYYPDPVLEPLSPTGLLELKPSSPLILKGRNLLPPAPGNSRLNYTVLIGST
PCTLTVSETQLLCEAPNLTGQHKVTVRAGGFEFSPGTLQVYSDSLLTLPAIVGIGGGGGLL
LLVIVAVLIAYKRKSRDADRTLKRLQLQMDNLESRVALECKEAFGGGGSDYKDHDGDYK
DHDIDYKDDDDKGGGGSGGGGSVSKGEELFTGVVPILVELDGDVNGHKFSVSGEGEGD
ATYGKLTLKFICTTGKLPVPWPTLVTTLTYGVQCFSRYPDHMKQHDFFKSAMPEGYVQE
RTIFFKDDGNYKTRAEVKFEGDTLVNRIELKGIDFKEDGNILGHKLEYNYNSHNVYIMAD
KQKNGIKVNFKIRHNIEDGSVQLADHYQQNTPIGDGPVLLPDNHYLSTQSALSKDPNEKR
DHMVLLEFVTAAGITLGMDELYK (SEQ ID NO: 17)
P324: eGFP-PLXNA1 (863-1300)-Flag-nanoluc; the fusion protein sequence is as follows:

MCHQQLVISWFSLVFLASPLVAVSKGEELFTGVVPILVELDGDVNGHKFSVSGEGEGDAT
YGKLTLKFICTTGKLPVPWPTLVTTLTYGVQCFSRYPDHMKQHDFFKSAMPEGYVQERTI
FFKDDGNYKTRAEVKFEGDTLVNRIELKGIDFKEDGNILGHKLEYNYNSHNVYIMADKQ
KNGIKVNFKIRHNIEDGSVQLADHYQQNTPIGDGPVLLPDNHYLSTQSALSKDPNEKRDH
MVLLEFVTAAGITLGMDELYKGGGGSGGGGSDPKILKLSPETGPRQGGTRLTITGENLGL
RFEDVRLGVRVGKVLCSPVESEYISAEQIVCEIGDASSVRAHDALVEVCVRDCSPHYRAL
SPKRFTFVTPTFYRVSPSRGPLSGGTWIGIEGSHLNAGSDVAVSVGGRPCSFSWRNSREIRC
LTPPGQSPGSAPIIININRAQLTNPEVKYNYTEDPTILRIDPEWSINSGGTLLTVTGTNLATV
REPRIRAKYGGIERENGCLVYNDTTMVCRAPSVANPVRSPPELGERPDELGFVMDNVRSL
LVLNSTSFLYYPDPVLEPLSPTGLLELKPSSPLILKGRNLLPPAPGNSRLNYTVLIGSTPCTL
TVSETQLLCEAPNLTGQHKVTVRAGGFEFSPGTLQVYSDSLLTLPAIVGIGGGGGLLLLVI

VAVLIAYKRKSRDADRTLKRLQLQMDNLESRVALECKEAFGGGGSDYKDHDGDYKDHD
IDYKDDDDKGGGGSGGGGSMVFTLEDFVGDWRQTAGYNLDQVLEQGGVSSLFQNLGV
SVTPIQRIVLSGENGLKIDIHVIIPYEGLSGDQMGQIEKIFKVVYPVDDHHFKVILHYGTLVI
DGVTPNMIDYFGRPYEGIAVFDGKKITVTGTLWNGNKIIDERLINPDGSLLFRVTINGVTG
WRLCERILA (SEQ ID NO: 18)

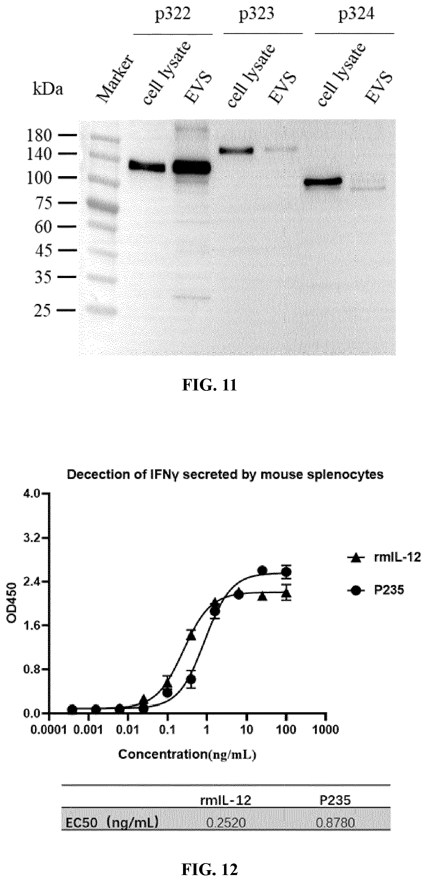

The WB detection results are shown in FIG. 11. Each fusion protein was successfully detected in EVs components.

### Example 9: Detection of the biological activity of the target protein in the fusion protein

In order to verify that the fusion-expressed extracellular protein (POI) still has biological activity, monocytes PBMC were extracted from the spleen of C57BL/6 mice. The P235 plasmid was transiently transfected into HEK293 cells, EVs were extracted from the supernatant, and the IL12 content of the EVs was quantified using ELISA. The *in vitro* activity of P235 EVs was tested using mIL12 recombinant protein as a control. The results are shown in FIG. 12. mIL12 fused and expressed with the truncated PLXNA1 also showed active function.

### Example 10: Detection of the biological activity of the target protein in the intracellular region of the fusion protein

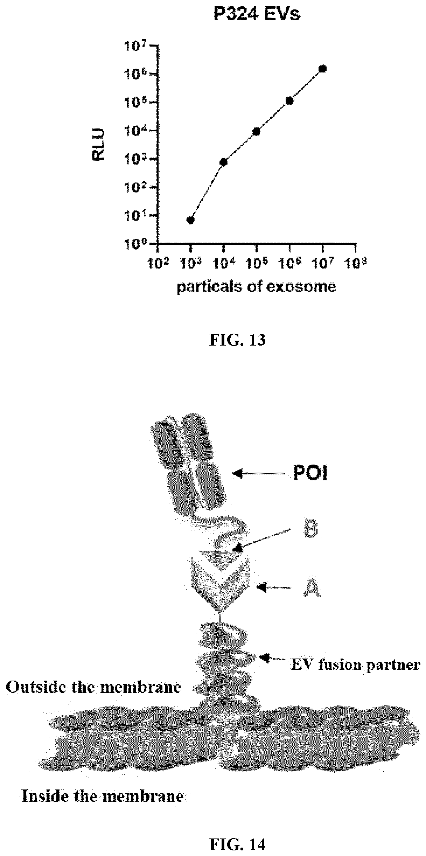

In order to verify that the target protein expressed in the intracellular region of the fusion protein still has biological activity, P324 EVs were extracted (see Example 8, P324: eGFP-PLXNA1 (863-1300)-Flag-nanoluc), and EVs with different particle numbers were detected for luciferase activity. 10 µl of EVs sample was taken, and then added with 10 µl of fluorescent substrate working solution, in which the substrate (Nano-Glo^{®} Luciferase Assay Substrate) was diluted 1:50 times with the matching buffer (Nano-Glo^{®} Luciferase Assay Buffer). After the reaction was performed for 5 minutes, the luminescence value was read with the microplate reader. The results are shown in FIG. 13. There is a good linear relationship between luciferase activity and the number of EVs particles.

### Example 11: Loading POI through NbALFA/ALFA interaction via EV fusion partner of the present invention

The above examples have confirmed that the EV fusion partner provided by the present invention can load the target protein fused therewith to EV. The target protein can be an affinity tag, and by connecting a part which binds to the affinity tag to a target molecule, the target molecule of interest can be loaded on the EV surface. That is, only a single type of engineered EV is produced, and through *in vitro* co-incubation with target molecules labeled with different specific tags, rapid loading of target molecules can be achieved, and EVs loaded with different proteins can be prepared. See FIG. 14 for a schematic diagram, wherein A and B are molecules that can bind to each other through non-covalent bonds.

### I. Construction of NbALFA-PLXΔ engineered extracellular vesicles (NbALFA-PLXΔ-EV)

NbALFA/ALFA tag is an affinity combination known in the art (Hansjörg Götzke., et al., 2019, Nat. Commun.; EP3849996A1). The PLXΔ sequence is the same as the P292 (863-1316) fragment sequence SEQ ID NO: 2 described in Example 3.

Plasmid construction: plasmid was designed to express fusion protein NbALFA-Flag-PLXΔ.

EV preparation: the plasmid was transfected into HEK293 to construct mixed clone cells. The cells were cultured, and the cell supernatant was collected to extract EV (also called NbALFA-PLXΔ-EV or NbALFA EV herein), and the cell lysate was collected for later use.

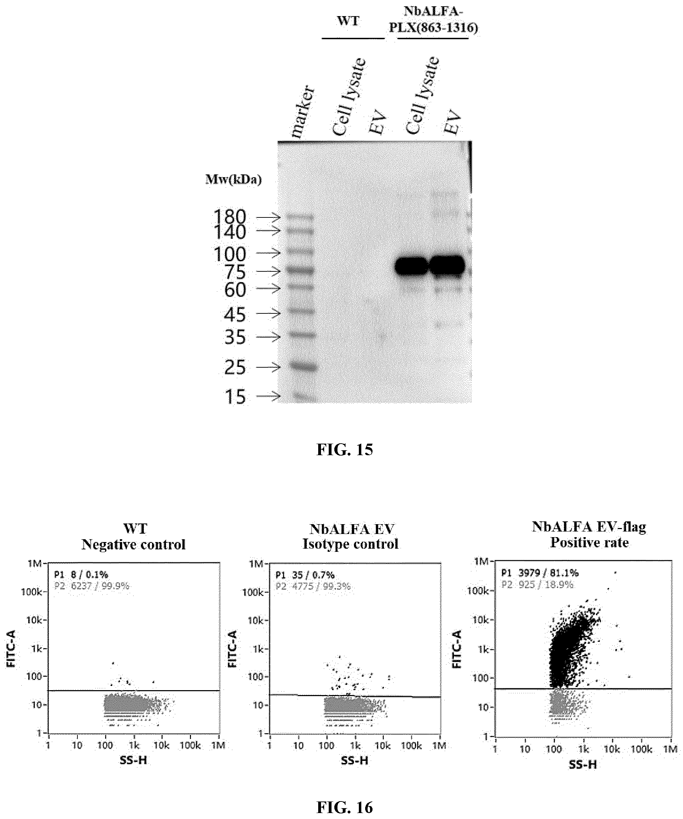

WB detection: NbALFA EVs and EVs of wild type 293 control were loaded at equal protein amount to perform WB experiments, and the flag expression was analyzed. The results are shown in FIG. 15;
Nanoflow cytometry: NbALFA EV was labeled with FITC-coupled anti-flag antibody, and the flag positive rate was detected through nanoflow cytometry. The results are shown in FIG. 16, in which the NbALFA EV isotype control is a homologous isotype FITC-conjugated antibody that does not specifically bind to flag, so that the non-specific effects of antibodies were eliminated. The specific isotype antibody used is mouse IgG2b type: FITC Plus Mouse IgG2b Isotype Control (Proteintech/ FITC-65128).

As can be seen from FIG. 15, no flag signal was detected in the wild-type 293 (WT) cell lysate and EV, but the flag signal was detected in the cell lysate and EV transfected with NbALFA-Flag-PLXΔ, indicating that PLXΔ was enriched on EVs. As can be seen from FIG. 16, the positive rate of NbALFA EV is greater than 70%.

### II. Loading GFP to NbALFA-PLXΔ-EV

GFP-ALFA preparation: plasmid was constructed, and ALFA-coupled GFP recombinant protein was expressed in prokaryotical cells, and quantified.

EV preparation: mixed clone cells expressing NbALFA-PLXΔ were cultured, the cell supernatant was collected to extract EVs, and wild type 293 EVs were used as a negative control.

Nanoflow cytometry: EV particle concentration was detected using Nanoflow cytometry.

*In vitro* labeling: in 100 µL PBS reaction system, 1E+9 NbALFA-PLXA-EV, 1% EV-free serum (FBS) and GFP-ALFA protein molecules (the ratio of the number of molecules to EV particles was 100: 1, 1000:1 and 10000:1) were added. Then the mixture was mixed thoroughly and incubated at 25°C for 30 minutes. Excess molecules were removed by size exclusion.

Nanoflow cytometry: the average fluorescence intensity of GFP was detected using nanoflow cytometry.

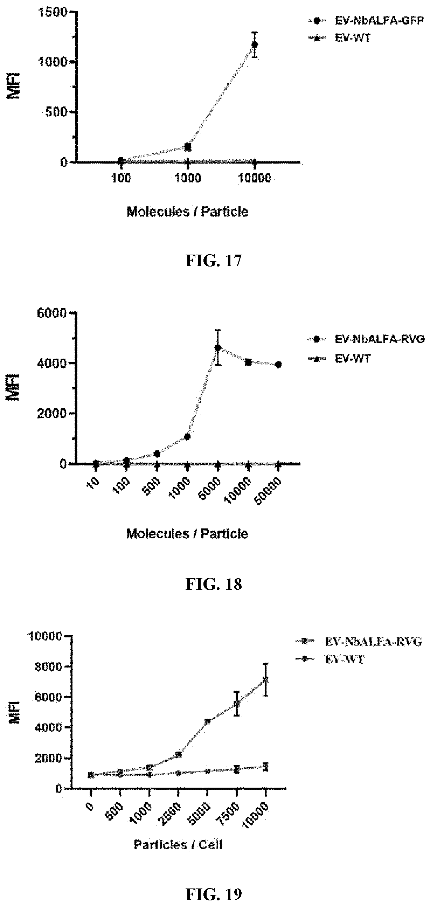

The results are shown in FIG. 17. GFP signal was detected on the EV surface, indicating that GFP-ALFA was effectively loaded onto NbALFA-PLXA-EV, and the fluorescence intensity increases with the number of GFP molecules increases.

### III. Loading RVG polypeptide to NbALFA-PLXΔ-EV

Preparation of RVG-HA-ALFA polypeptide: RVG-HA-ALFA was synthesized and quantified.

EV preparation: mixed clone cells expressing NbALFA-PLXΔ were cultured, the cell supernatant was collected to extract EVs, and wild type 293 EV was used as a negative control.

Nanoflow cytometry: EV particle concentration was detected using nanoflow cytometry.

*In vitro* labeling: in 100 µL PBS reaction system, 1E+9 NbALFA-PLXA-EV, 1% EV-free serum (FBS) and RVG-HA-ALFA polypeptide molecules (the ratio of the number of molecules to EV particles were respectively 10:1, 100:1, 500:1, 1000:1, 5000:1, 10000:1 and 50000:1) were added. Then the mixture was mixed thoroughly and incubated at 25°C for 30 minutes. Excess molecules were removed by size exclusion.

Nanoflow cytometry: EVs loading RVG were labeled with FITC-coupled anti-HA antibody, and the average fluorescence intensity of HA was detected through nanoflow cytometry.

The results are shown in FIG. 18. Compared with the negative control, RVG-ALFA can be effectively labeled on NbALFA-PLXA-EV, and it tended to saturate labeling when the feeding ratio reached 5000.

Targeting activity detection: RVG-labeled vesicles were stained and labeled with lipophilic green fluorescent dye CMG (CellMask^{™} Green Plasma Membrane Stain, Invitrogen/C37608), and then co-incubated with mouse neuroma blast cell line (N2a). The input ratio of the number of extracellular vesicle particles to the number of cells was set to be 0, 500, 1000, 2500, 5000, 7500 and 10000 respectively. The mixture was incubated in a cell culture incubator for 2 hours, then washed with PBS to remove excess vesicles, and the CMG fluorescence intensity was detected using flow cytometry. The results are shown in FIG. 19. Compared with the wild-type control group, RVG labeled vesicles had significantly stronger affinity for recipient N2a cells.

### IV. Loading trastuzumab scFv fragment to NbALFA-PLXΔ-EV

scFv-HA-ALFA polypeptide synthesis: plasmid was constructed, and ALFA-coupled scFv recombinant protein (ALFA is located at the C-terminus of scFv) was expressed in eukaryotic cells and quantified.

EV preparation: mixed clone cells expressing NbALFA-PLXΔ were cultured, the cell supernatant was collected to extract EVs, and wild type 293 EV was used as a negative control.

Nanoflow cytometry: EV particle concentration was detected using nanoflow cytometry.

*In vitro* labeling: in 1000 µL. PBS reaction system, 1E+11 NbALFA-PLXΔ-EV, 1% EV-free serum (FBS) and scFv-HA-ALFA protein molecules (the ratio of the number of molecules to EV particles was 10000 :1) were added. Then the mixture was mixed thoroughly and incubated at 25°C for 30 minutes. Excess molecules were removed by size exclusion.

Nanoflow cytometry results: scFv loaded EVs were labeled with FITC-coupled anti-HA antibody, and the average fluorescence intensity of HA was detected by nanoflow cytometry.

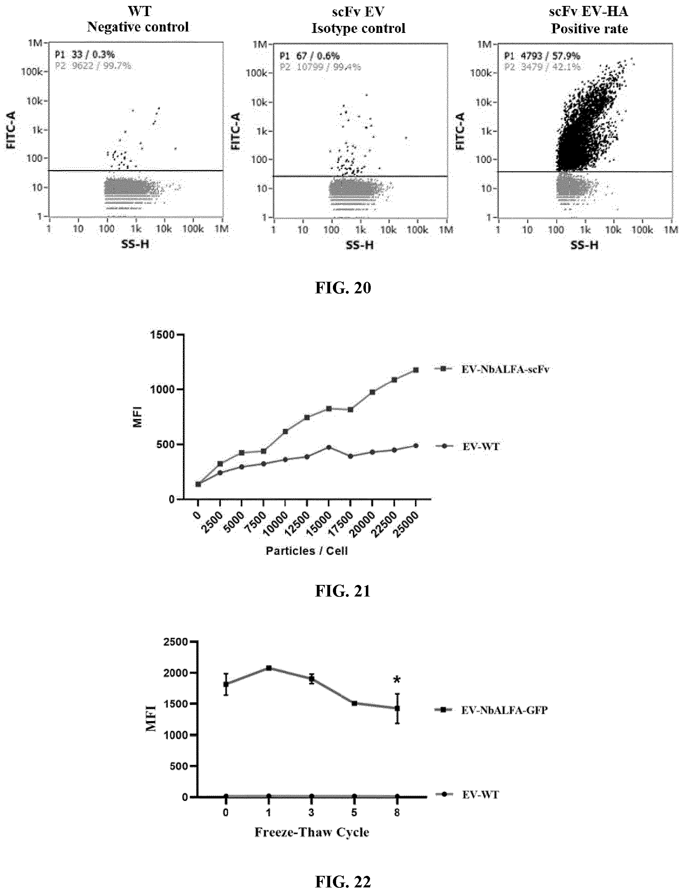

The results are shown in FIG. 20. Compared with the negative control and isotype control (mouse IgG1 isotype control monoclonal antibody, Proteintech/66360-1-Ig), scFv-ALFA was effectively labeled to NbALFA-PLXA-EV.

Targeting activity detection: scFv-labeled vesicles were stained with lipophilic green fluorescent dye CMG, and co-incubated with breast cancer cell line (SK-BR-3). The input ratios of the number of extracellular vesicle particles to the number of cells were set to be 0, 2500:1, 5000:1, 7500:1, 10000:1, 12500:1, 15000:1, 17500:1, 20000:1, 22500:1 and 25000:1 respectively. The mixture was incubated in a cell incubator for 2 hours, and then washed with PBS to remove excess vesicles, and CMG fluorescence intensity was detected using flow cytometry. The results are shown in FIG. 21. Compared with the wild-type control group, the scFv labeled vesicles had stronger affinity for the recipient SK-BR-3 cells.

### V. NbALFA-PLXΔ engineered extracellular vesicles labeled with target molecules have good stability

*In vitro* labeling NbALFA-PLXΔ-EV with GFP-ALFA: *in vitro* labeling experiments was performed according to the method described in Part I, and the average fluorescence intensity of GFP was detected using nanoflow cytometry.

Freeze-thaw cycle test: GFP-labeled NbALFA-PLXΔ-EV was left at -80°C, frozen completely (about 15 minutes), then taken out and left to stand at room temperature to thaw. This was recorded as one freeze-thaw cycle. 8 freeze-thaw cycles were completed according to this method. The average fluorescence intensity of GFP after the 1st, 3rd, 5th and 8th freeze-thaw cycle was detected using nanoflow cytometry.

The results are shown in FIG. 22. Before the 8th freeze-thaw cycle, the labeled molecules were stably bound, and the fluorescence of the labeled molecules only decreased slightly at the 8th freeze-thaw cycle.

Storage condition test: GFP-tagged NbALFA-PLXΔ-EV was stored at 4°C or -80°C, taken out at the 2nd and 4th weeks respectively, and left to stand at room temperature for 1 hour, and then the average fluorescence intensity of GFP was detected using nanoflow cytometry.

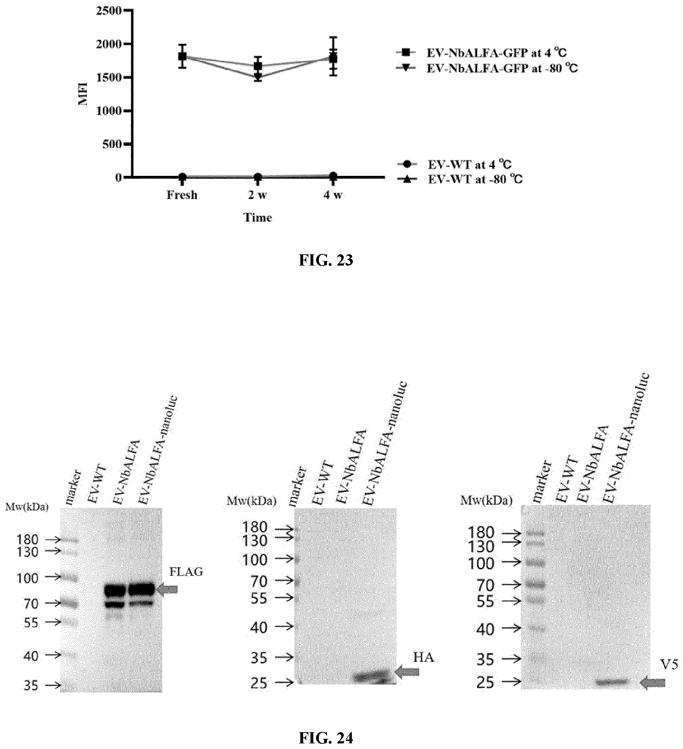

The results are shown in FIG. 23. The binding of the labeled molecules are stable during 4 weeks of storage at 4°C or -80°C.

### Example 12: Labeling EVs with different protein combinations

NbALFA-PLXΔ-EVs were simultaneously labeled with nanoluc-HA-ALFA and nanoluc-V5-ALFA *in vitro*: nanoluc-HA-ALFA and nanoluc-V5-ALFA were mixed with equal numbers of molecules, the *in vitro* labeling experiment was performed according to the previous method, and WB detection was performed using HA tag antibody and V5 tag antibody respectively. When multiple proteins were used for *in vitro* labeling, the ratio of various proteins could be adjusted as needed. The WB results are shown in FIG. 24, showing that target molecules carrying different tags were simultaneously labeled on the vesicles.

### Example 13: Loading target protein (POI) through biotin/avidin interaction via EV fusion partner of the present invention

### I. Construction of mSA-PLXΔ engineered extracellular vesicles (mSA-PLXΔ-EV)

Plasmid construction: plasmid was designed to express the fusion protein mSA-Flag-PLXΔ, mSA is the abbreviation of monomer strepavidin.

EV preparation: the plasmid was transfected into HEK293 to construct mixed clone cells. The cells were cultured, the cell supernatant was collected to extract EV (also called mSA-PLXΔ-EV or mSAEV herein), and the cell lysate was collected for later use.

WB detection: mSA EVs and wild type 293 control EVs were loaded with equal protein amounts to perform WB experiments, and the expression of flag was analyzed.

Nanoflow cytometry: mSA EVs were labeled with FITC-coupled anti-flag antibody, and the flag positivity rate was detected using nanoflow cytometry.

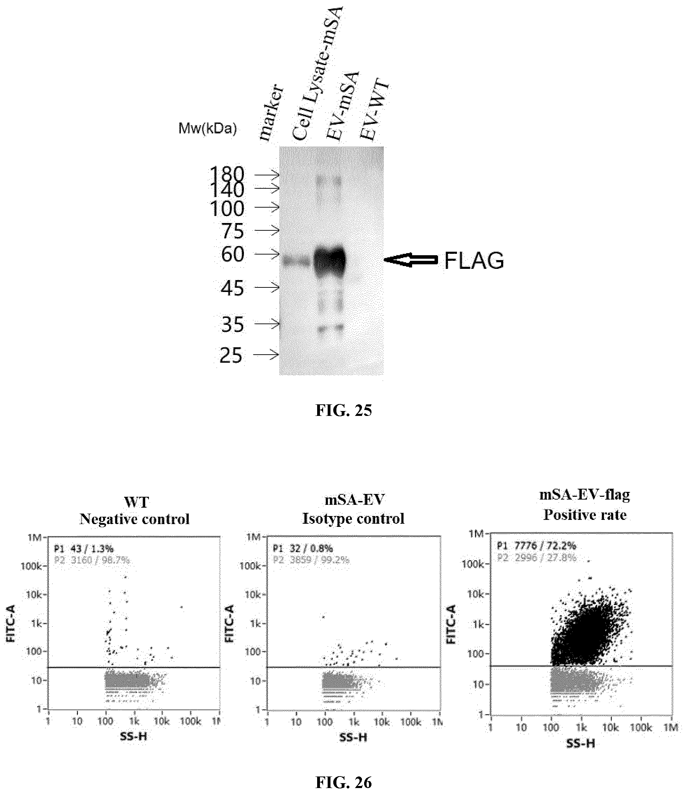

The WB results are shown in FIG. 25. No flag signal was detected in the wild-type 293 (WT) cell lysate and EV, but the flag signal could be detected in the cell lysate and EVs transfected with mSA-Flag-PLXΔ, indicating that PLXΔ was enriched on EVs. The nanoflow cytometry results are shown in FIG. 26. It can be seen that the mSA EV positive rate exceeded 70%.

### II. Loading biotin modified molecules to mSA-PLXΔ engineered extracellular vesicles

EV preparation: mixed clone cells expressing mSA-PLXΔ were cultured, and the cell supernatant was collected to extract EVs. Wild type 293 EVs were used as a negative control.

Nanoflow cytometry: EV particle concentration was detected using nanoflow cytometry.

*In vitro* labeling: in 100 µL PBS reaction system, 1E+9 mSA-PLXΔ-EV, 1% EV-free serum (FBS) and Alexa Fluor 488-PEG3K-biotin molecules (the ratio of the number of molecules and EV particles was 10,000:1) were added. Then the mixture was mixed thoroughly and incubated at 25°C for 30 minutes. Excess molecules were removed by size exclusion.

Nanoflow cytometry: Alexa Fluor 488 fluorescence positivity rate was detected using nanoflow cytometry.

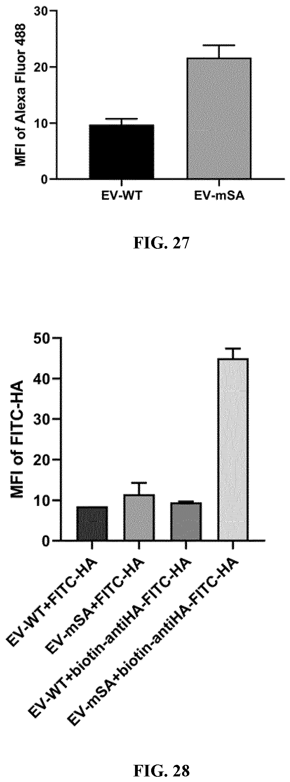

The results are shown in FIG. 27. Compared with the negative control, molecules with biotin-488 were labeled on mSA-PLXA-EV.

### III. Loading biotin-modified HA antibody to mSA-PLXΔ-EV

EV preparation: mixed clone cells expressing mSA-PLXΔ were cultured, and the cell supernatant was collected to extract EVs. Wild type 293 EVs were used as a negative control.

Nanoflow cytometry: EV particle concentration was detected using nanoflow cytometry.

*In vitro* labeling: in 100 µL PBS reaction system, 1E+9 mSA-PLXΔ-EV, 1% EV-free serum (FBS) and biotin-modified HA antibody (Invitrogen/26183-BTIN) (the ratio of the number of molecules and EV particles was 10,000:1) were added. Then the mixture was mixed thoroughly and incubated at 25°C for 30 minutes. Excess molecules were removed by size exclusion.

Binding FITC-coupled HA polypeptide (Ruixibio/R-YAF-010): the above HA antibody-labeled vesicles and FITC-coupled HA polypeptide (the ratio of the number of molecules and EV particles was 10,000:1) were mixed and incubated at 25°C for 30 minutes, and excess molecules were removed by size exclusion. FITC fluorescence positivity rate was detected by nanoflow cytometry.

The results are shown in FIG. 28. Based on the affinity of mSA-biotin, vesicles labeled with anti-HA antibodies were labeled with FITC-HA.

### Example 14: Loading target protein (POI) through strepII/streptactin interaction via the EV fusion partner of the present invention

FITC-streptactin expression: A plasmid was constructed, streptactin was prokaryotically expressed, and labeled with FITC fluorescent molecules.

EV preparation: The strepII-flag-PLXΔ plasmid was designed and transfected into HEK293 to construct mixed clone cells. The cells were cultured, the cell supernatant was collected to extract EVs, and the cell lysate was collected for later use. Wild type 293 EV was used as a negative control.

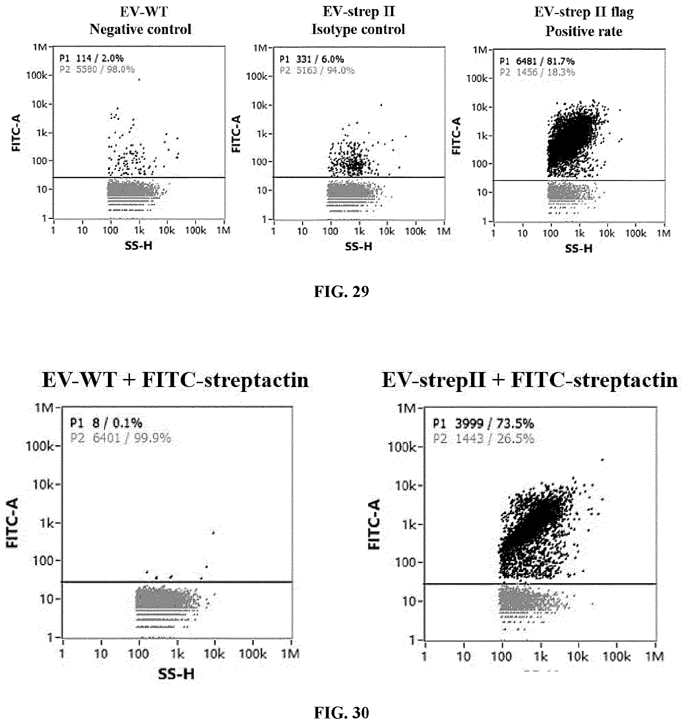

Nanoflow cytometry: EV particle concentration and flag positive rate was detected using nanoflow cytometry. The results are shown in FIG. 29. The strepII-flag-PLXΔ-EV positive rate exceeded 80%;
*In vitro* labeling: in 100 µL PBS reaction system, 1E+9 mSA-PLXΔ vesicles, 1% EV-free serum (FBS) and FITC-streptactin molecules (the ratio of the number of molecules and EV particles was 10,000:1) were added. Then the mixture was mixed thoroughly and incubated at 25°C for 30 minutes.

FITC detection: GFP was detected using nanoflow cytometry. The results are shown in FIG. 30. FITC-streptactin was effectively labeled on EVs, and the positive rate reached 73%.

All publications and patents mentioned in the present application are incorporated by reference. Without departing from the scope and spirit of the present invention, various modifications and variations of the described methods and compositions of the present invention will be apparent to those skilled in the art. Although the present invention has been described in terms of specific preferred embodiments, it should be understood that the present invention as claimed should not be unduly limited to these specific embodiments. Indeed, various variations of the described modes for carrying out the present invention that are obvious to those skilled in the relevant art are intended to be included within the scope of the appended claims.

## Claims

1. An engineered extracellular vesicle comprising a fusion protein, wherein the fusion protein comprises a fusion partner, which is a protein located on the extracellular vesicle membrane or a variant thereof, preferably, the fusion protein further comprises a target protein.

2. The extracellular vesicle according to claim 1, wherein the fusion partner is **characterized in that**: 1) the fusion partner is a type I transmembrane protein; 2) a transmembrane region and adjacent intracellular region of the transmembrane protein comprise an α-helix in a length of 20-200 amino acids, preferably 30-90 amino acids, more preferably 40-80 amino acids, and part or all of the transmembrane region is located in the α-helix; and 3) the extracellular region comprises 1 to 5 IPT domains.

3. The extracellular vesicle according to claim 1 or 2, wherein the fusion partner is selected from the group consisting of PLXNA, PLXNB, PLXNC and PLXND family members, and a variant thereof.

4. The extracellular vesicle according to claim 3, wherein the fusion partner is selected from the group consisting of PLXNA1, PLXNA2, PLXNA3, PLXNA4, PLXNB1, PLXNB2, PLXNC1, PLXND1, and a variant thereof, preferably PLXNA1, PLXNA2 and a variant thereof.

5. The extracellular vesicle according to claim 4, wherein the fusion partner comprises a sequence selected from the group consisting of SEQ ID NOs: 2-6.

6. The extracellular vesicle according to any one of claims 1 to 5, wherein the target protein is located upstream or downstream of the fusion partner.

7. The extracellular vesicle according to any one of claims 1 to 6, wherein the target protein is a therapeutic peptide, a targeting peptide, an affinity tag or a linker linking a therapeutic compound.

8. The extracellular vesicle according to claim 7, wherein the therapeutic peptide is an antibody and/or a cytokine, for example, the cytokine is selected from the group consisting of a member of the human interleukin family (e.g., IL-2, IL-7, IL-10, IL-11, IL-12, IL-15, and IL-23), a member of the tumor necrosis factor family (e.g., TNF, LTA, LTB, FASLG, TNFSF8, TNFSF9, TNFSF10, TNFSF11, TNFSF12, TNFSF13, TNFSF14, TNFSF15, TNFSF18 and EDA), an interferon (INF-α, INF-β and INF-γ), a T cell engager (e.g., 4-1BB, OX40, CD28, CD40, CD40L, CD47, CD27, CD70, CD80, CD86, GITRL, ICOSL, CD155, CD112, TIM-3, BTLA), and other cytokines (e.g., G-CSF, EPO, TPO, GM-CSF, EGF, bFGF, FVIIa, ATIII, TNK, α-Glucosidase, BMP-2, hirudin).

9. The extracellular vesicle according to claim 7, wherein the targeting peptide is a cell-targeting polypeptide fragment selected from the group consisting of an antibody, an antigen-binding fragment thereof, a cell surface receptor, and a ligand thereof.

10. The extracellular vesicle according to claim 7, wherein the affinity tag is selected from the group consisting of His tag, glutathione sulfhydryl transferase (GST), S-peptide, ZZ domain, albumin binding domain (ABD), HA, Myc, FLAGTM, maltose-binding protein (MBP), calmodulin-binding peptide (CBP), SUMO, *Streptococcus* protein G (Protein G) and *Staphylococcus aureus* protein A (Protein A).

11. The extracellular vesicle according to any one of claims 1 to 10, comprising two or more fusion proteins, for example, the two or more fusion proteins each independently comprise different target proteins.

12. The extracellular vesicle according to any one of claims 1 to 11, comprising a therapeutic agent, and the therapeutic agent is selected from the group consisting of a therapeutic peptide, a polynucleotide and a small molecule compound.

13. A pharmaceutical composition comprising the extracellular vesicle according to any one of claims 1 to 12 and a pharmaceutically acceptable carrier.

14. An engineered cell for producing the extracellular vesicle according to any one of claims 1 to 12.

15. The cell according to claim 14, comprising a polynucleotide encoding the fusion protein, preferably the polynucleotide is integrated in the genome of the cell.

16. A method of treating a disease, comprising administering to a subject in need thereof the extracellular vesicle according to any one of claims 1 to 12, the pharmaceutical composition according to claim 13, or the cell according to claim 14.

17. A method of producing the extracellular vesicle according to any one of claims 1 to 12, comprising
1) providing a cell comprising a polynucleotide encoding the fusion protein,
2) culturing the cell under a condition suitable for expression of the fusion protein, and
2) isolating the extracellular vesicle.

18. A method of delivering a target protein to a target cell, comprising
1) providing the extracellular vesicle according to any one of claims 1 to 12, wherein the fusion protein comprises a target protein to be delivered, and
2) contacting the extracellular vesicle with the target cell.

19. A method of isolating the extracellular vesicle according to any one of claims 1 to 12, comprising
1) causing cells capable of expressing the fusion protein to secrete the extracellular vesicle, wherein the fusion protein comprises an affinity tag,
2) contacting the extracellular vesicle with a binding agent capable of binding the affinity tag, and
3) isolating the extracellular vesicle based on the binding of the affinity tag to the binding agent.

20. A fusion protein, which is an engineered transmembrane protein expressed from an exogenous sequence, and the engineered transmembrane protein comprises a fragment represented by formula (I),
Xaa₁...Xaaₙ Xaa₍ₙ₊₁₎Xaa₍ₙ₊₂₎Xaa₍ₙ₊₃₎Xaa₍ₙ₊₄₎ (I), (SEQ ID NO: 15)
preferably, the fragment represented by formula (I) is located upstream of the transmembrane region,
wherein, Xaa₁ is selected from the group consisting of S, T, C, N, Y and Q; Xaa₂ to Xaaₙ are any amino acids, n is an integer from 1 to 20; Xaa₍ₙ₊₁₎, Xaa₍ₙ₊₂₎ and Xaa₍ₙ₊₄₎ are each independently selected from the group consisting of L, I, G, A and V; Xaa₍ₙ₊₃₎ is selected from the group consisting of T, S, C, N, Y and Q.

21. The fusion protein according to claim 20, wherein n is 1, 2, 3, 4, 5, 6, 7, 8, 9, 10, 11, 12, 13, 14, 15, 16, 17, 18, 19 or 20, preferably 1, 2, 3, 4, 5, 6, 7, 8, 9 or 10, more preferably 1, 2, 3, 4 or 5.

22. The fusion protein according to claim 20 or 21, wherein Xₐₐ₁ is selected from the group consisting of S, T, C and N; Xaa₍ₙ₊₁₎, Xaa₍ₙ₊₂₎ and Xaa₍ₙ₊₄₎ are each independently selected from the group consisting of L and I; Xaa₍ₙ₊₃₎ is selected from the group consisting of T, S, C and N.

23. The fusion protein according to claim 22, wherein Xₐₐ₁ is S, Xaa₍ₙ₊₁₎, Xaa₍ₙ₊₂₎ and Xaa₍ₙ₊₄₎ are L, and Xaa₍ₙ₊₃₎ is T.

24. The fusion protein according to claim 23, wherein the fragment represented by formula (I) is SDSLLTL (SEQ ID NO: 14).

25. The fusion protein according to any one of claims 20-24, comprising a sequence selected from the group consisting of: SEQ ID NOs: 2-6.

26. A polynucleotide encoding the fusion protein according to any one of claims 20-25.

27. An engineered extracellular vesicle comprising the fusion protein according to any one of claims 20-25.

28. A method of loading a target molecule to the extracellular vesicle according to any one of claims 1 to 12, comprising contacting the target molecule with the extracellular vesicle, wherein the target molecule and the fusion partner are connected to a part A and a part B respectively, and the part A and part B are bound by affinity.

29. The method according to claim 28, wherein the part A and part B are a combination selected from the group consisting of NbALFA/ALFA, biotin/avidin, strepII/streptactin, Intein N/C, SpyCatcher/Spy and Protein A/Fc.

30. The method according to claim 28 or 29, wherein the target molecule is a therapeutic peptide, a targeting peptide, an affinity tag or a linker linking a therapeutic compound.
